# EUROPEAN PATENT APPLICATION

(11) **EP 3 719 113 A1**
(43) Date of publication of application: **07.10.2020**
(21) Application number: 18883582.1
(22) Date of filing: 29.11.2018
(51) Int. Cl.: C12M 3/00, C12N 5/00, C09D 185/02, C09D 201/06

(54) **CELL CULTURE CONTAINER CAPABLE OF LONG-TERM CULTURE, AND METHOD FOR MANUFACTURING SAME**

(30) Priority: 29.11.2017 JP 2017228902
(71) Applicant: Nissan Chemical Corporation, Tokyo 103-6119 (JP)
(72) Inventor: SUZUKI Kohei, Funabashi-shi Chiba 274-0052 (JP); HIROI Yoshiomi, Funabashi-shi Chiba 274-0052 (JP); IWAKAMI Masashi, Shiraoka-shi Saitama 349-0294 (JP); NISHINO Taito, Shiraoka-shi Saitama 349-0294 (JP)
(74) Representative: dompatent von Kreisler Selting Werner - Partnerschaft von Patent- und Rechtsanwälten mbB
(86) International application number: PCT/JP2018/044014
(87) International publication number: WO 2019/107503

(57) **Abstract**

The present invention provides a cell culture container for long term non-adherent cell culture which comprises having a coating containing a copolymer which is a copolymer having a recurring unit which contains a group represented by the following formula (a), a recurring unit which contains a group represented by the following formula (b), and a recurring unit which contains a group represented by the following formula (c), and a molar ratio of the recurring unit which contains a group represented by the formula (c) being 1 to 50 mol% based on the whole copolymer, provided with at least a part of a surface thereof.

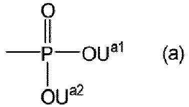

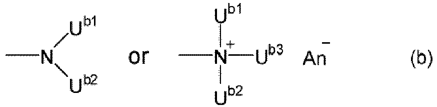

-R^{c} (c)

[wherein, U^{a1}, U^{a2}, U^{b1}, U^{b2} and U^{b3}, R^{c}, and An⁻ are as defined in the specification and Claims.]

## Description

### TECHNICAL FIELD

The present invention relates to a cell culture container for long term non-adherent cell culture and a method for manufacturing the same, and a method for manufacturing cell aggregates using the same.

### BACKGROUND ART

In recent years, technologies to proliferate or maintain various organs, tissues and cells that play different roles in animals and plants *in vitro* have been developed. To proliferate or maintain these organs and tissues *in vitro* is called organ culture and tissue culture, respectively, and to proliferate, differentiate or maintain cells separated from organs and tissues *in vitro* is called cell culture. The cell culture is a technology for proliferating, differentiating or maintaining isolated cells in a medium *in vitro,* and is indispensable for analyzing functions and structures of various organs, tissues and cells *in vivo* in detail. In addition, the cells and/or tissues cultured by the technology are utilized in various fields such as medical efficacy and toxicity evaluation of chemical substances, pharmaceuticals, etc., mass production of useful substances such as enzymes, cell growth factors, antibodies, etc., regenerative medicine to supplement organs, tissues and cells lost by diseases or defects, selective breeding of plants, creation of genetically recombined crops, and the like.

For culture of cells, cell culture containers such as schale (petri dishes), dishes, flasks, bags, plates, chamber slides, tubes, trays, bottles, etc., are generally used, and are appropriately selected depending on various conditions such as a kind of cells, culture method, culture scale, etc. The material of cell culture container is exclusively glass or a resin and, for example, when the cell culture container is made of a resin and the surface thereof is hydrophobic, there is a problem that adhesion of cells and components indispensable for cell culture such as cell growth factors, etc., to the surface of the container occurs whereby proliferative and expression of functions of cells are impaired.

Different from conventional two-dimensional monolayer culture, as a culturing method for constructing a three-dimensional structure of cells *in vitro,* a method of forming cell aggregates (also called as cell spheroid, cell cluster and the like) has attracted attention. It has been reported that cell aggregates are three-dimensionally cultured, which are cell gathered materials in which cells are gathered and aggregated and a living body-like structure is constructed, so that functions of the cells can be maintained for a long term and physiological functions are improved. Therefore, expectations of the cell aggregates for utilization in drug discovery research, or in cell therapy or regenerative therapy is increasing. Accompanied thereby, a demand of a cell culture container in which adhesion of cells or cell aggregates to the surface of the container is inhibited and suitable for three-dimensional culture is increasing.

For example, it has been reported that as a method for culturing cells in a non-adhered state, a material selected from agar, agarose and poly(2-hydroxyethyl methacrylate) is used as a substrate, and cancer cells are cultured on the substrate in a non-adhered state for the maximum of 12 days to form cell aggregates (for example, see Patent Document 1). Also, it has been reported that as a use when cultured as cell aggregates, a protein which causes tumor formation is identified by culturing cancer cells isolated from a patient as cell aggregates in a cell non-adhered state and a mechanism of canceration is elucidated (for example, see Non-Patent Document 1).

On the other hand, it has been known that a material having, at the surface thereof, a polymer material containing cations and anions in the side chain has a function of preventing adsorption of a biological substance (proteins, cells, etc.) since the surface thereof is maintained to electrically neutral due to the electrostatic balance. Also, it has been proposed a coating material using these functions, and various reports have been made on the methods of adhering and fixing to a glass and polymer substrate, etc. For example, the present inventors have focused on a polymer having a phosphoric acid ester group which is expected to be a coating material having a function of inhibiting adhesion of various biological substances and earnestly studied. As a result, they have reported that a coating agent containing a copolymer which contains a specific anionic group and cationic group can be firmly adhered irrespective of the kind of the substrate, and after adhesion, it becomes a coating film excellent in resistance to an aqueous solvent and exhibits excellent function to inhibit adhesion of biological substances (for example, platelets, fibrinogen, etc.) (for example, see Patent Documents 2 and 3). In addition, it has also been reported that a cell culture container which comprises being coated with the coating agent is excellent in cell-adhesion inhibiting function and also excellent in resistance to solvents and radiation (for example, see Patent Document 4).

### PRIOR ART DOCUMENTS

### PATENT DOCUMENTS

Patent Document 1: JP 2017-60498A
Patent Document 2: WO 2014/196650
Patent Document 3: WO 2016/093293
Patent Document 4: WO 2014/196652

### NON-PATENT DOCUMENT

Non-Patent Document 1: Todaro M el al. Cell Stem Cell 4, 389-402 (2007)

### SUMMARY OF THE INVENTION

### PROBLEMS TO BE SOLVED BY THE INVENTION

Two-dimensional monolayer culture is generally carried out by seeding cells in a monolayer state onto a scaffold material like an extracellular matrix or onto a culture container physically subjected to surface treatment. On the other hand, three-dimensional culture for forming cell aggregates is generally carried out by subjecting to suspension culture of cells using a cell culture container having a non-adhesive surface. In particular, in order to obtain a large amount of cell aggregates having a uniform shape and size stably and easily, it is desirable to carry out culture for a long term, but such a cell culture container capable of realizing such a non-adhesive culture of the cells for a long term has not been reported.

### MEANS TO SOLVE THE PROBLEMS

The present inventors have found that a cell culture container provided with a coating which contains a copolymer containing a specific anionic structure, a specific cationic structure and a specific hydrophobic structure, and existing a hydrophobic structure with a specific ratio, at least a part of the surface of the container, enables non-adhesion culture of the cells for a long term, whereby they have accomplished the present invention.

The present invention is as follows.
[1] A cell culture container for long term non-adherent cell culture which comprises having a coating containing a copolymer which is a copolymer having a recurring unit which contains a group represented by the following formula (a), a recurring unit which contains a group represented by the following formula (b), and a recurring unit which contains a group represented by the following formula (c):

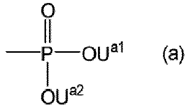

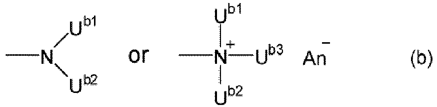

   -R^{c} (c)

   [wherein
   U^{a1}, U^{a2}, U^{b1}, U^{b2} and U^{b3} each independently represent a hydrogen atom or a linear or branched alkyl group having 1 to 5 carbon atoms,
   R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)), and
   An⁻ represents an anion selected from the group consisting of a halide ion, an inorganic acid ion, a hydroxide ion and an isothiocyanate ion], and
   a molar ratio of the recurring unit which contains a group represented by the formula (c) being 1 to 50 mol% based on the whole copolymer, provided with at least a part of a surface thereof.
[2] The cell culture container described in the above-mentioned [1], wherein the recurring units containing groups represented by the above-mentioned formulae (a), (b) and (c) are recurring unit derived from monomers represented by the following formulae (A), (B) and (C): 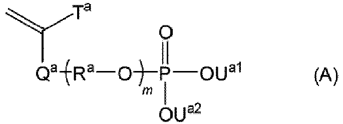 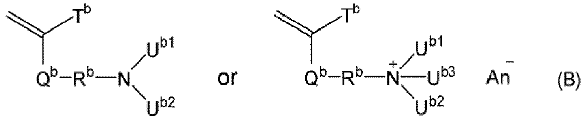 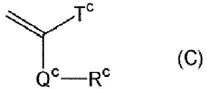 [wherein,
   T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms;
   Q^{a} and Q^{b} each independently represent a single bond, an ester bond or an amide bond, Q^{c} represents a single bond, an ether bond or an ester bond;
   R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s));
   An⁻ represents an anion selected from the group consisting of a halide ion, an inorganic acid ion, a hydroxide ion and an isothiocyanate ion; and
   m represents an integer of 0 to 6],
   respectively.
[3] The cell culture container described in the above-mentioned [1] or [2], wherein the copolymer further contains a crosslinking structure derived from a monomer represented by the following formula (D) or (E): 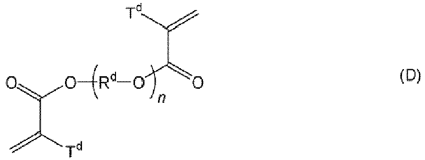 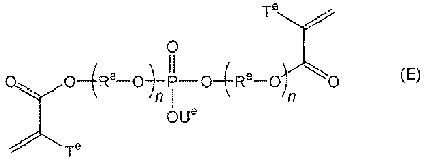 [wherein,
   T^{d}, T^{e} and U^{e} each independently represent a hydrogen atom or a linear or branched alkyl group having 1 to 5 carbon atoms,
   R^{d} and R^{e} each independently represent a linear or branched alkylene group having 1 to 10 carbon atoms which may be substituted by a halogen atom(s); and n represents an integer of 1 to 6].
[4] The cell culture container described in the above-mentioned any of [1] to [3], which has a function of inhibiting adhesion of a biological substance.
[5] A method for manufacturing a cell culture container for long term non-adherent cell culture which comprises a step of coating a copolymer containing a recurring unit which contains a group represented by the following formula (a), a recurring unit which contains a group represented by the following formula (b), and a recurring unit which contains a group represented by the following formula (c):

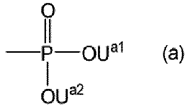

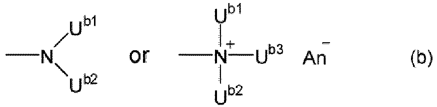

   -R^{c} (c)

   [wherein,
   U^{a1}, U^{a2}, U^{b1}, U^{b2} and U^{b3} each independently represent a hydrogen atom or a linear or branched alkyl group having 1 to 5 carbon atoms;
   R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)); and
   An⁻ represents an anion selected from the group consisting of a halide ion, an inorganic acid ion, a hydroxide ion and an isothiocyanate ion],
   and a molar ratio of the recurring unit which contains a group represented by the formula (c) being 1 to 50 mol% based on a whole copolymer, onto at least a part of a surface of the container.
[6] The manufacturing method described in the above-mentioned [5], wherein the recurring units containing groups represented by the above-mentioned formulae (a), (b) and (c) are recurring units derived from monomers represented the following formulae (A), (B) and (C): 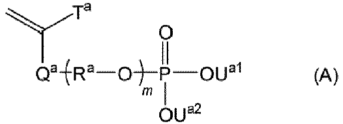 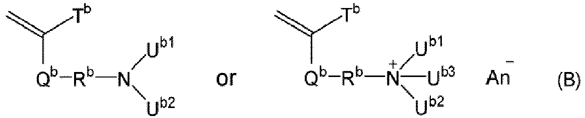 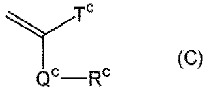 [wherein,
   T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms;
   Q^{a} and Q^{b} each independently represent a single bond, an ester bond or an amide bond, Q^{c} represents a single bond, an ether bond or an ester bond;
   R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)); An⁻ represents an anion selected from the group consisting of a halide ion, an inorganic acid ion, a hydroxide ion and an isothiocyanate ion; and
   m represents an integer of 0 to 6],
   respectively.
[7] The manufacturing method described in the above-mentioned [5] or [6], wherein the coating step is carried out by using a varnish containing the copolymer.
[8] The manufacturing method described in the above-mentioned [7], wherein a pH of the varnish containing the copolymer is adjusted in advance.
[9] The manufacturing method described in the above-mentioned any one of [5] to [8], which further comprises a step of washing the coated cell culture container before and/or after a drying step.
[10] The manufacturing method described in the above-mentioned [9], wherein the washing after the drying step is carried out by using at least one solvent selected from the group consisting of water and an aqueous solution containing an electrolyte(s).
[11] A method for manufacturing cell aggregates which comprises using the cell culture container described in the above-mentioned any one of [1] to [4] or the cell culture container manufactured by the manufacturing method described in the above-mentioned any one of [5] to [10].

### EFFECTS OF THE INVENTION

The cell culture container of the present invention is provided with a coating containing a copolymer which contains an anionic group represented by the formula (a), a cationic group represented by the formula (b) and a hydrophobic group represented by the formula (c), and the hydrophobic group represented by the formula (c) being present with a specific ratio, at least a part of the surface of the container, so that it can be used for non-adhesion culture for a long term (for example, 14 days or longer, for example, 21 days or longer).

### BRIEF DESCRIPTION OF THE DRAWINGS

Fig. 1 is a photomicrograph showing a state of cell adhesion after 4 days of culture of a plate of each Example and Comparative Example, and a plate of positive control and negative control, which were subjected to a fibroblast cell adhesion inhibition test of Test Example 2.
Fig. 2 is a photomicrograph showing a state of cell adhesion before removing a medium after 21 days of culture of plates of each Example and Comparative Example, and plates of a positive control and a negative control applied to a cancer cell adhesion inhibition test of Test Example 3.
Fig. 3 is a photomicrograph showing a state of cell adhesion after removing a medium and staining after 21 days of culture of plates of each Example and Comparative Example, and plates of a positive control and a negative control applied to a cancer cell adhesion inhibition test of Test Example 3.
Fig. 4 is a schematic drawing of a PDMS coating plate used in Test Example 5.
Fig. 5 is an enlarged view of a dimple portion and a cross section of a PDMS coating plate used in Test Example 5.
Fig. 6 is a schematic drawing of a production scheme of a PDMS coating plate used in Test Example 5.
Fig. 7 is a photomicrograph showing a state of cell adhesion after 1 day of culture of plates of each Example and Comparative Example and plates of a negative control applied to a cell adhesion inhibition test of Test Example 5.

### EMBODIMENTS TO CARRY OUT THE INVENTION

### «Explanation of the terms»

The terms used in the present invention have the following definitions, otherwise specifically mentioned.

In the present invention, the "halogen atom" means a fluorine atom, a chlorine atom, a bromine atom or an iodine atom.

In the present invention, the "alkyl group" means a linear or branched, saturated monovalent aliphatic hydrocarbon group. The "linear or branched alkyl group having 1 to 5 carbon atoms" may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. The "linear or branched alkyl group having 1 to 10 carbon atoms" may be mentioned, in addition to the examples of the "linear or branched alkyl group having 1 to 5 carbon atoms", a hexyl group, a heptyl group, an octyl group, a nonyl group or a decyl group, or an isomer thereof.

Among these, a methyl group, an ethyl group, an n-propyl group, an isopropyl group, an n-butyl group, an isobutyl group, an s-butyl group and a t-butyl group are preferable.

In the present invention, the "linear or branched alkyl group having 1 to 5 carbon atoms which may be substituted by a halogen atom(s)" means either the above-mentioned linear or branched alkyl group having 1 to 5 carbon atoms, or the above-mentioned linear or branched alkyl group having 1 to 5 carbon atoms substituted by one or more of the above-mentioned halogen atoms. Examples of the "linear or branched alkyl group having 1 to 5 carbon atoms" are as mentioned above. On the other hand, the "linear or branched alkyl group having 1 to 5 carbon atoms substituted by one or more halogen atoms" means a group in which one or more optional hydrogen atoms of the above-mentioned linear or branched alkyl group having 1 to 5 carbon atoms is/are substituted by a halogen atom(s), and examples thereof may be mentioned a fluoromethyl group, a difluoromethyl group, a trifluoromethyl group, a chloromethyl group, a dichloromethyl group, a trichloromethyl group, a bromomethyl group, an iodomethyl group, a 2,2,2-trifluoroethyl group, a 2,2,2-trichloroethyl group, a perfluoroethyl group, a perfluorobutyl group or a perfluoropentyl group, etc.

In the present invention, the "ester bond" means -C(=O)-O- or -O-C(=O)-, the "amide bond" means -NHC(=O)- or -C(=O)NH- and the "ether bond" means -O-.

In the present invention, the "linear or branched alkylene group having 1 to 10 carbon atoms which may be substituted by a halogen atom(s)" means a linear or branched alkylene group having 1 to 10 carbon atoms or a linear or branched alkylene group having 1 to 10 carbon atoms substituted by one or more halogen atoms. Here, the "alkylene group" means a divalent organic group corresponding to the above-mentioned alkyl group. Examples of the "linear or branched alkylene group having 1 to 10 carbon atoms" may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, a hexamethylene group, an octamethylene group and a decamethylene group, etc., among these, an ethylene group, a propylene group, an octamethylene group and a decamethylene group are preferred, and, for example, a linear or branched alkylene group having 1 to 5 carbon atoms such as an ethylene group, a propylene group, a trimethylene group, a tetramethylene group, etc., are more preferred, and, in particular, an ethylene group or a propylene group is preferred. The "linear or branched alkylene group having 1 to 10 carbon atoms substituted by one or more halogen atoms" means a group in which one or more optional hydrogen atoms of the above-mentioned alkylene group is/are substituted by a halogen atom(s), and, in particular, a part or whole of the hydrogen atom(s) of the ethylene group or the propylene group is/are substituted by a halogen atom(s) is/are preferred.

In the present invention, the "alicyclic hydrocarbon group having 3 to 10 carbon atoms" means a monocyclic or polycyclic, saturated or partially unsaturated, monovalent aliphatic hydrocarbon group having 3 to 10 carbon atoms. Among these, a monocyclic or bicyclic, saturated monovalent aliphatic hydrocarbon group having 3 to 10 carbon atoms is preferred, and there may be mentioned, for example, a cycloalkyl group having 3 to 10 carbon atoms such as a cyclopropyl group, a cyclobutyl group and a cyclohexyl group, etc., or a bicycloalkyl group having 4 to 10 carbon atoms such as a bicyclo[3.2.1]octyl group, a bornyl group and an isobornyl group, etc.

In the present invention, the "aryl group having 6 to 10 carbon atoms" means a monocyclic or polycyclic, monovalent aromatic hydrocarbon group having 6 to 10 carbon atoms, and there may be mentioned, for example, a phenyl group, a naphthyl group or an anthryl group, etc. The "aryl group having 6 to 10 carbon atoms" may be substituted by one or more of the above-mentioned "linear or branched alkyl group having 1 to 5 carbon atoms which may be substituted by a halogen atom(s)."

In the present invention, the "aralkyl group having 7 to 15 carbon atoms" means a group -R-R' (here, R represents the above-mentioned "alkylene group having 1 to 5 carbon atoms", and R' represents the above-mentioned "aryl group having 6 to 10 carbon atoms"), and there may be mentioned, for example, a benzyl group, a phenethyl group or an α-methylbenzyl group, etc. The aryl portion of the "aralkyl group having 7 to 15 carbon atoms" may be substituted by one or more of the above-mentioned "linear or branched alkyl group having 1 to 5 carbon atoms which may be substituted by a halogen atom(s)."

In the present invention, the "aryloxyalkyl group having 7 to 15 carbon atoms" means a group -R-O-R' (here, R represents the above-mentioned alkylene group having 1 to 5 carbon atoms", and R' represents the above-mentioned "aryl group having 6 to 10 carbon atoms"), and there may be mentioned, for example, a phenoxymethyl group, a phenoxyethyl group or a phenoxypropyl group, etc. The aryl portion of the "aryloxyalkyl group having 7 to 15 carbon atoms" may be substituted by one or more of the above-mentioned "linear or branched alkyl group having 1 to 5 carbon atoms which may be substituted by a halogen atom(s)."

In the present invention, "a halide ion" means a fluoride ion, a chloride ion, a bromide ion or an iodide ion.

In the present invention, "an inorganic acid ion" means a carbonate ion, a sulfate ion, a phosphate ion, a hydrogen phosphate ion, a dihydrogen phosphate ion, a nitrate ion, a perchlorate ion or a borate ion.

As the above-mentioned An⁻, preferred are a halide ion, a sulfate ion, a phosphate ion, a hydroxide ion and an isothiocyanate ion, and particularly preferred is a halide ion.

In the present invention, the (meth)acrylate compound means both of an acrylate compound and a methacrylate compound. For example, the (meth)acrylic acid means acrylic acid and methacrylic acid.

In addition, in the present invention, the "anion" or the "anionic group" means a negative ion or a negative ionic group, and also contains a group capable of becoming a negative ion or a negative ionic group by dissociating in water. Similarly, in the present invention, the "cation" or the "cationic group" means a positive ion or a positive ionic group, and also contains a group capable of becoming a positive ion or a positive ionic group by dissociating in water.

In the present invention, the biological substance may be mentioned a peptide, a protein, a saccharide, a nucleic acid and a cell or a combination thereof. The peptide or protein may be mentioned, for example, fibrinogen, bovine serum albumin (BSA), human albumin, various kinds of globulins, β-lipoprotein, various kinds of antibodies (IgG, IgA, IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen and cytochrome c, the saccharide may be mentioned, for example, glucose, galactose, mannose, fructose, heparin and hyaluronic acid, the nucleic acid may be mentioned, for example, deoxyribonucleic acid (DNA) and ribonucleic acid (RNA), the cell may be mentioned, for example, fibroblast, bone marrow cells, B lymphocytes, T lymphocytes, neutrophils, red blood cells, platelets, macrophages, monocytes, bone cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells, mononuclear cells, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells and various kinds of cell lines (for example, HCT116, Huh7, HEK293 (human embryonic kidney cell), HeLa (human cervical cancer cell lines), HepG2 (human liver cancer cell lines), UT7/TPO (human leukemia cell lines), CHO (Chinese hamster ovary cell lines), MDCK, MDBK, BHK, C-33A, HT-29, AE-1, 3D9, Ns0/1, Jurkat, NIH3T3, PC12, S2, Sf9, Sf21, High Five, Vero), etc.,

Having a function to inhibit adhesion of the biological substance means that, for example,
when the biological substance is a protein, it means that a mass (%) per a relative unit area ((a mass per a unit area of Example (ng/cm²)/(a mass per a unit area of Comparative Example (ng/cm²))) when compared with no coating film by the QCM-D measurement carried out by the method described in Example is 50% or less, preferably 30% or less, and further preferably 20% or less; or
when the biological substance is a protein, it means that a relative optical density (450nm) (%) ((optical density (450nm) of Example)/(optical density (450nm) of Comparative Example)) when compared with no coating film by the plate reader carried out by the method described in Example is 50% or less, preferably 30% or less, further preferably 20% or less, and further preferably 10% or less;
when the biological substance is a cell, it means that a relative absorbance (WST O.D. 450 nm) (%) ((absorbance of Example (WST O.D. 450 nm))/(absorbance of Comparative Example (WST O.D. 450 nm))) when compared with no coating film by the fluorescence microscope carried out by the method described in Example is 50% or less, preferably 30% or less, further preferably 20% or less, further preferably 10% or less, and most preferably 6% or less.

In the present invention, long term cell non-adhesion culture means that in a cell culture requiring a time of a long term (for example, 14 days or longer, or 21 days or longer), the cells can be cultured in a non-adhesion state. Further, due to this effect, a force to adhere the cells with each other becomes stronger than a force to adhere the cells to the container, and depending on a cell species, formation of a cell aggregate (spheroid) can be efficiently carried out.

In a general culture container, the adhesive force of the cells to the substrate is usually designed to be larger than the adhesive force between the cells, so that it is possible to culture the cells by adhering to the substrate, but by coating the coating agent of the present application to the substrate, the adhesive force between the cells becomes larger than the adhesive force of the cells to the substrate so that a spheroid can be formed.

### «Explanation of the present invention»

The cell culture container for long term non-adherent cell culture of the present invention comprises having a coating containing a copolymer which is a copolymer having a recurring unit which contains a group represented by the following formula (a), a recurring unit which contains a group represented by the following formula (b), and a recurring unit which contains a group represented by the following formula (c):

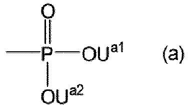

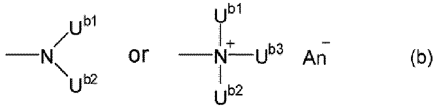

-R^{c} (c)

[wherein, U^{a1}, U^{a2}, U^{b1}, U^{b2} and U^{b3} each independently represent a hydrogen atom or a linear or branched alkyl group having 1 to 5 carbon atoms; R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)); and An⁻ represents an anion selected from the group consisting of a halide ion, an inorganic acid ion, a hydroxide ion and an isothiocyanate ion], and a molar ratio of the recurring unit which contains a group represented by the formula (c) being 1 to 50 mol% based on the whole copolymer, provided with at least a part of a surface thereof.

The copolymer according to the present invention is not particularly limited as long as it is a copolymer containing a recurring unit which contains a group represented by the above-mentioned formula (a), a recurring unit which contains a group represented by the above-mentioned formula (b) and a recurring unit which contains a group represented by the above-mentioned formula (c), and a molar ratio of the recurring unit containing a group represented by the formula (c) is 1 to 50 mol% based on the whole copolymer. Incidentally, in the present invention, the recurring unit which contains a group represented by the above-mentioned formula (c) is different from the recurring unit which contains a group represented by the above-mentioned formula (a) and the recurring unit which contains a group represented by the above-mentioned formula (b). The polymer is desirably a material obtained by radical polymerizing a monomer containing a group represented by the above-mentioned formula (a), a monomer containing a group represented by the above-mentioned formula (b) and a monomer containing a group represented by the above-mentioned formula (c), but a material obtained by subjecting to polycondensation or polyaddition reaction may be also used. Examples of the copolymer may be mentioned a vinyl polymerized polymer in which olefins are reacted, polyamide, polyester, polycarbonate, polyurethane, etc., and among these, a vinyl polymerized polymer in which olefins are reacted or a (meth)acrylic polymer in which a (meth)acrylate compound(s) is/are polymerized is desirable.

A ratio of the recurring unit which contains a group represented by the formula (a) in the copolymer according to the present invention is 3 mol% to 80 mol%, preferably 5 mol% to 60 mol%, and further preferably 7 mol% to 40 mol%. It is most preferably 10 mol% to 30 mol%. Incidentally, the copolymer according to the present invention may contain two or more kinds of the recurring units which contain a group represented by the formula (a).

A ratio of the recurring unit which contains a group represented by the formula (b) in the copolymer according to the present invention is 3 mol% to 80 mol%, preferably 10 mol% to 75 mol%, and further preferably 20 mol% to 70 mol%. It is most preferably 40 mol% to 65 mol%. Incidentally, the copolymer according to the present invention may contain two or more kinds of the recurring units which contain a group represented by the formula (b).

A ratio of the recurring unit containing a group represented by the formula (c) in the copolymer according to the present invention is 1 mol% to 50 mol%, and preferably 3 mol% to 45 mol%. It is further preferably 5 mol% to 40 mol%. It is most preferably 10 mol% to 35 mol%. Incidentally, the copolymer according to the present invention may contain two or more kinds of the recurring units which contain a group represented by the formula (c).

A combination of a ratio of the recurring units which contain groups represented by the above-mentioned formula (a), the formula (b) and the formula (c) in the copolymer according to the present invention is preferably,
3 mol% to 80 mol% of the formula (a), 3 mol% to 80 mol% of the formula (b), and 1 mol% to 50 mol% of the formula (c),
   more preferably,
5 mol% to 60 mol% of the formula (a), 10 mol% to 75 mol% of the formula (b), and 3 mol% to 45 mol% of the formula (c),
   further preferably,
7 mol% to 40 mol% of the formula (a), 20 mol% to 70 mol% of the formula (b), and 5 mol% to 40 mol% of the formula (c),
   and most preferably,
10 mol% to 30 mol% of the formula (a), 40 mol% to 65 mol% of the formula (b), and 10 mol% to 35 mol% of the formula (c).

As the copolymer according to the present invention, a copolymer containing the recurring units of the following formulae (a1), (b1) and (c1) is particularly preferably used.

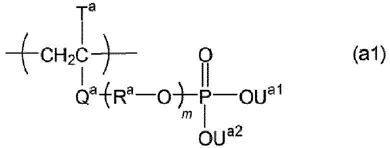

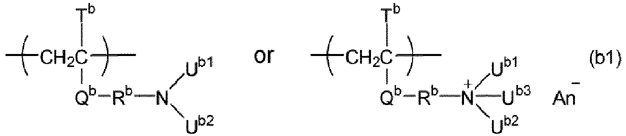

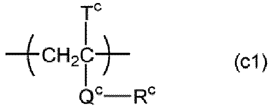

In the formula, T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms, Q^{a} and Q^{b} each independently represent a single bond, an ester bond or an amide bond, Q^{c} represents a single bond, an ether bond or an ester bond, R^{a} and R^{b} each independently represent a linear or branched alkylene group having 1 to 10 carbon atoms which may be substituted by a halogen atom(s), R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s)), An⁻ represents an anion selected from the group consisting of a halide ion, an inorganic acid ion, a hydroxide ion and an isothiocyanate ion, m represents an integer of 0 to 6.

In the formula (a1), m represents an integer of 0 to 6, preferably represents an integer of 1 to 6, more preferably represents an integer of 1 to 5, and is particularly preferably 1.

A ratio of the recurring unit represented by the formula (a1) contained in the copolymer according to the present invention is 3 mol% to 80 mol%, preferably 5 mol% to 60 mol%, further preferably 7 mol% to 40 mol%, and most preferably 10 mol% to 30 mol%. Incidentally, the copolymer according to the present invention may contain two or more kinds of the recurring units represented by the formula (a1).

A ratio of the recurring unit represented by the formula (b 1) contained in the copolymer according to the present invention is 3 mol% to 80 mol%, preferably 10 mol% to 75 mol%, further preferably 20 mol% to 70 mol%, and most preferably 40 mol% to 65 mol%. Incidentally, the copolymer according to the present invention may contain two or more kinds of the recurring units represented by the formula (b1).

A ratio of the recurring unit represented by the formula (c1) contained in the copolymer according to the present invention is 1 mol% to 50 mol%, preferably 3 mol% to 45 mol%, further preferably 5 mol% to 40 mol%, and most preferably 10 mol% to 35 mol%. Incidentally, the copolymer according to the present invention may contain two or more kinds of the recurring units represented by the formula (c1).

A combination of a ratio of the recurring units represented by the above-mentioned formula (a1), the formula (b1) and the formula (c1) in the copolymer according to the present invention is
preferably
3 mol% to 80 mol% of the formula (a1), 3 mol% to 80 mol% of the formula (b1), and 1 mol% to 50 mol% of the formula (c1),
   more preferably
5 mol% to 60 mol% of the formula (a1), 10 mol% to 75 mol% of the formula (b1), and 3 mol% to 45 mol% of the formula (c1),
   further preferably
7 mol% to 40 mol% of the formula (a1), 20 mol% to 70 mol% of the formula (b1), and 5 mol% to 40 mol% of the formula (c1), and
   most preferably,
10 mol% to 30 mol% of the formula (a1), 40 mol% to 65 mol% of the formula (b1), and 10 mol% to 35 mol% of the formula (c1).

The copolymer according to the present invention can be also obtained by reacting (polymerizing) a monomer mixture containing compounds represented by the following formulae (A), (B) and (C): 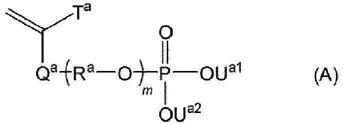 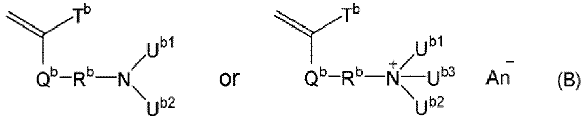 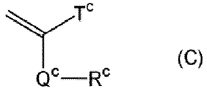 [wherein,
T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms;
Q^{a} and Q^{b} each independently represent a single bond, an ester bond or an amide bond, Q^{c} represents a single bond, an ether bond or an ester bond;
R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (here, the above-mentioned aryl portion may be substituted by a linear or branched alkyl group having 1 to 5 carbon atoms which may be substituted by a halogen atom(s));
An⁻ represents an anion selected from the group consisting of a halide ion, an inorganic acid ion, a hydroxide ion and an isothiocyanate ion;
m represents an integer of 0 to 6]
in a solvent.

T^{a}, T^{b} and T^{c} are preferably a hydrogen atom, a methyl group or an ethyl group, more preferably a hydrogen atom or a methyl group. U^{a1}, U^{a2}, U^{b1}, U^{b2} and U^{b3} are preferably a hydrogen atom, a methyl group, an ethyl group or a t-butyl group, U^{a1} and U^{a2} of the formula (a) is more preferably a hydrogen atom, and U^{b1}, U^{b2} and U^{b3} of the formula (b) are more preferably a hydrogen atom, a methyl group, an ethyl group or a t-butyl group.

Specific examples of the above-mentioned formula (A) may be mentioned vinylphosphonic acid, acid phosphoxyethyl (meth)acrylate, 3-chloro-2-acid phosphoxypropyl (meth)acrylate, acid phosphoxypropyl (meth)acrylate, acid phosphoxymethyl (meth)acrylate, acid phosphoxypolyoxyethylene glycol mono(meth)acrylate, acid phosphoxypolyoxypropylene glycol mono(meth)acrylate, etc., among these, vinylphosphonic acid, acid phosphoxyethyl methacrylate (=2-(methacryloyloxy)ethyl phosphate), acid phosphoxypolyoxyethylene glycol monomethacrylate and acid phosphoxypolyoxypropylene glycol monomethacrylate are preferably used.

The structural formulae of vinylphosphonic acid, acid phosphoxyethyl methacrylate (=2-(methacryloyloxy)ethyl phosphate), acid phosphoxypolyoxyethylene glycol monomethacrylate and acid phosphoxypolyoxypropylene glycol monomethacrylate are represented by the following formula (A-1) to the formula (A-4), respectively.

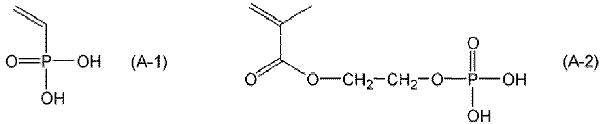

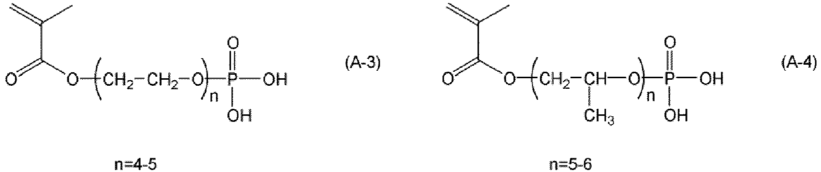

For example, acid phosphoxyethyl methacrylate (=phosphoric acid 2-(methacryloyloxy)ethyl) is a compound contained in the product name; Phosmer M (available from Unichemical Co.) and LIGHT ESTER P-1M (available from KYOEISHA CHEMICAL Co., Ltd.).

For example, acid phosphoxypolyoxyethylene glycol monomethacrylate is a compound contained in the product name; Phosmer PE (available from Unichemical Co.).

For example, acid phosphoxypolyoxypropylene glycol monomethacrylate is a compound contained in the product name; Phosmer PP (available from Unichemical Co.).

These compounds may contain a (meth)acrylate compound having two functional groups as represented by the general formula (D) or (E) mentioned later at the time of synthesis in some cases.

Specific examples of the above-mentioned formula (B) may be mentioned dimethylaminoethyl (meth)acrylate, diethylaminoethyl (meth)acrylate, dimethylaminopropyl (meth)acrylate, 2-(t-butylamino)ethyl (meth)acrylate, methacryloylcholine chloride, etc., among these, dimethylaminoethyl (meth)acrylate, methacryloylcholine chloride or 2-(t-butylamino)ethyl (meth)acrylate is preferably used.

The structural formulae of dimethylaminoethyl acrylate (=acrylic acid 2-(dimethylamino)ethyl), diethylaminoethyl methacrylate (=methacrylic acid 2-(diethylamino)ethyl), dimethylaminoethyl methacrylate (=methacrylic acid 2-(dimethylamino)ethyl), methacryloylcholine chloride and 2-(t-butylamino)ethyl methacrylate (=methacrylic acid 2-(t-butylamino)ethyl) are represented by the following formula (B-1) to the formula (B-5), respectively.

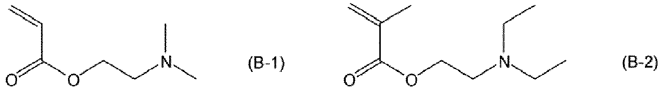

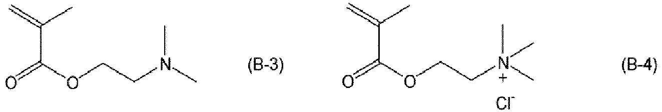

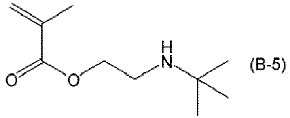

Specific examples of the above-mentioned formula (C) may be mentioned linear or branched alkyl esters of (meth)acrylic acid such as methyl (meth)acrylate, ethyl (meth)acrylate, n-propyl (meth)acrylate, isopropyl (meth)acrylate, n-butyl (meth)acrylate, isobutyl (meth)acrylate, s-butyl (meth)acrylate, t-butyl (meth)acrylate, hexyl (meth)acrylate, 2-ethylhexyl (meth)acrylate, etc.; cyclic alkyl esters of (meth)acrylic acid such as cyclohexyl (meth)acrylate, isobornyl (meth)acrylate, etc.; aralkyl esters of (meth)acrylic acid such as benzyl (meth)acrylate, phenethyl (meth)acrylate, etc.; styrene-based monomers such as styrene, methylstyrene, chloromethylstyrene, etc.; vinyl ether-based monomers such as methyl vinyl ether, butyl vinyl ether, etc.; and vinyl ester-based monomers such as vinyl acetate, vinyl propionate, etc. Among these, linear or branched alkyl esters of (meth)acrylic acid having 1 to 6 carbon atoms such as methyl (meth)acrylate, ethyl (meth)acrylate, butyl (meth)acrylate, hexyl (meth)acrylate, etc., or cyclic alkyl esters of (meth)acrylic acid having 3 to 6 carbon atoms such as cyclohexyl (meth)acrylate, etc., are preferable, in particular, methyl (meth)acrylate, ethyl (meth)acrylate, n-propyl (meth)acrylate, isopropyl (meth)acrylate, n-butyl (meth)acrylate, isobutyl (meth)acrylate, s-butyl (meth)acrylate, t-butyl (meth)acrylate and n-hexyl (meth)acrylate are preferable, and methyl (meth)acrylate, ethyl (meth)acrylate, n-propyl (meth)acrylate, isopropyl (meth)acrylate, n-butyl (meth)acrylate, isobutyl (meth)acrylate, s-butyl (meth)acrylate and t-butyl (meth)acrylate are more preferable.

For example, the structural formulae of n-butyl methacrylate (=methacrylic acid n-butyl) and cyclohexyl methacrylate (=methacrylic acid cyclohexyl) are represented by the following formula (C-1) and the formula (C-2), respectively.

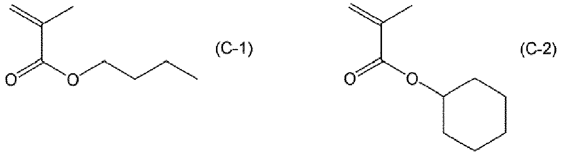

In the copolymer according to the present invention, an optional fourth component may be further copolymerized. For example, as the fourth component, a (meth)acrylate compound having two or more functional groups may be copolymerized, and a part of the polymer may be partially three-dimensionally crosslinked. Such a fourth component may be mentioned, for example, a bifunctional monomer represented by the following formula (D) or (E): 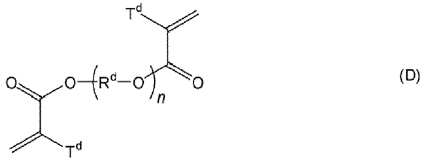 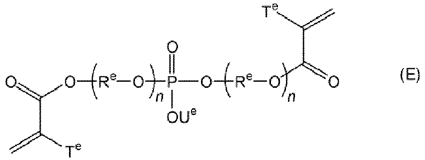 [wherein, T^{d}, T^{e} and U^{e} each independently represent a hydrogen atom or a linear or branched alkyl group having 1 to 5 carbon atoms, R^{d} and R^{e} each independently represent a linear or branched alkylene group having 1 to 10 carbon atoms which may be substituted by a halogen atom(s); and n represents an integer of 1 to 6]. That is, the copolymer according to the present invention preferably contains a crosslinking structure derived from such a bi-functional monomer.

In the formulae (D) and (E), T^{d} and T^{e} are preferably each independently a hydrogen atom, a methyl group or an ethyl group, and more preferably each independently a hydrogen atom or a methyl group.

In the formula (E), U^{e} is preferably a hydrogen atom, a methyl group or an ethyl group, and more preferably a hydrogen atom.

In the formula (D), R^{d} preferably represents a linear or branched alkylene group having 1 to 3 carbon atoms which may be substituted by a halogen atom(s), more preferably each independently represent an ethylene group or a propylene group, or an ethylene group or a propylene group substituted by one chlorine atom, and particularly preferably an ethylene group or a propylene group. In addition, in the formula (D), n preferably represents an integer of 1 to 5, and particularly preferably 1.

In the formula (E), R^{e} preferably represents a linear or branched alkylene group having 1 to 3 carbon atoms which may be substituted by a halogen atom(s), more preferably each independently represent an ethylene group or a propylene group, or an ethylene group or a propylene group substituted by one chlorine atom, and particularly preferably an ethylene group or a propylene group. Also, in the formula (E), n preferably represents an integer of 1 to 5, and particularly preferably 1.

In the formulae (D) and (E), preferable is the formula (E).

The bifunctional monomer represented by the formula (D) may be preferably mentioned ethylene glycol di(meth)acrylate, triethylene glycol di(meth)acrylate, and propylene glycol di(meth)acrylate, or a bifunctional monomer derived from the above-mentioned formula (A-3) or (A-4).

The bifunctional monomer represented by the formula (E) may be preferably mentioned bis(methacryloyloxymethyl) phosphate, bis[(2-methacryloyloxy)ethyl] phosphate, bis[3-(methacryloyloxy)propyl] phosphate, or a bifunctional monomer derived from the above-mentioned formula (A-3) or (A-4).

In addition, as the trifunctional (meth)acrylate compound, there may be mentioned phosphinylidynetris(oxy-2,1-ethanediyl) triacrylate.

Among these fourth components, in particular, among ethylene glycol di(meth)acrylate, propylene glycol di(meth)acrylate, bis[2-(methacryloyloxy)ethyl] phosphate, bis[3-(methacryloyloxy)propyl] phosphate and among the bi-functional monomer derived from the above-mentioned formulae (A-3) and (A-4), a di(meth)acrylate having a recurring unit of ethylene glycol or propylene glycol, and a di(meth)acrylate having a recurring unit of ethylene glycol or propylene glycol through a phosphate ester group are preferable, and the structural formulae thereof are represented by the following formulae (D-1) to (D-3) and formulae (E-1) to (E-3), respectively. In particular, the formulae (E-1) to (E-3) are preferable.

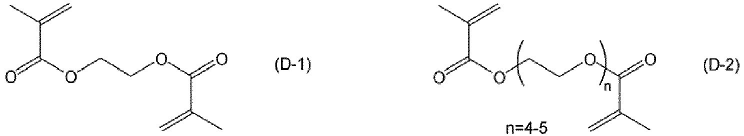

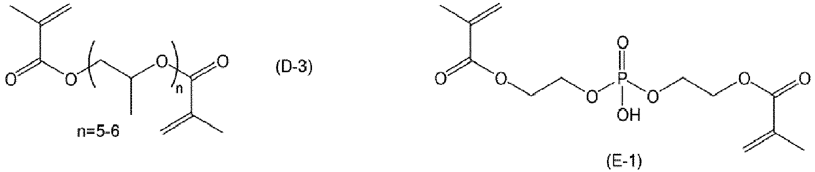

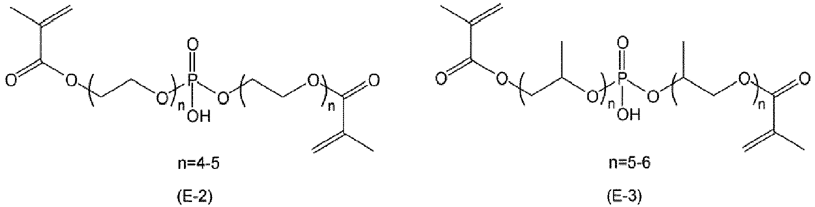

In the copolymer, one or two or more kinds of these fourth components may be contained.

A ratio of the fourth component in the above-mentioned copolymer, for example, a crosslinking structure derived from the bi-functional monomer represented by the above-mentioned formula (D) or (E) is 0 mol% to 50 mol%.

A ratio of the compound represented by the formula (A) in the whole monomer constituting the above-mentioned copolymer is 3 mol% to 80 mol%, preferably 5 mol% to 60 mol%, further preferably 7 mol% to 40 mol%, and most preferably 10 mol% to 30 mol%. In addition, the compound represented by the formula (A) may be two or more kinds.

A ratio of the compound represented by the formula (B) in the whole monomer constituting the above-mentioned copolymer is 3 mol% to 80 mol%, preferably 10 mol% to 75 mol%, further preferably 20 mol% to 70 mol%, and most preferably 40 mol% to 65 mol%. In addition, the compound represented by the formula (B) may be two or more kinds.

A ratio of the compound represented by the formula (C) in the whole monomer constituting the above-mentioned copolymer is 1 mol% to 50 mol%, preferably 3 mol% to 45 mol%, further preferably 5 mol% to 40 mol%, and most preferably 10 mol% to 35 mol%. In addition, the compound represented by the formula (C) may be two or more kinds.

In the copolymer according to the present invention, as a further optional fifth component, an ethylenically unsaturated monomer, or a polysaccharide or derivatives thereof may be further copolymerized. Examples of the ethylenically unsaturated monomer may be mentioned one or two or more kinds of the ethylenically unsaturated monomers selected from the group consisting of a (meth)acrylic acid and an ester thereof; vinyl acetate; vinylpyrrolidone; ethylene; vinyl alcohol; and a hydrophilic functional derivative thereof. Examples of the polysaccharides or derivatives thereof may be mentioned a cellulose-based polymer such as hydroxyalkyl cellulose (for example, hydroxyethyl cellulose or hydroxypropyl cellulose), etc., starch, dextran and curdlan.

Examples of the hydrophilic functional derivatives refer to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or a divalent group of a compound having an amphoteric center of a quaternary ammonium type cation structure and an acidic anion structure and may be mentioned, for example, a phosphorylcholine group: 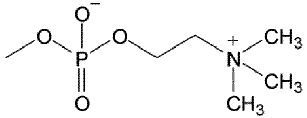 Examples of the ethylenically unsaturated monomer having such a structure may be mentioned 2-methacryloyloxyethylphosphorylcholine (MPC), etc.

The amide structure means a group represented by the following formula: 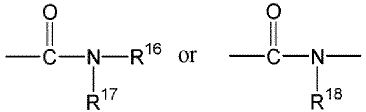 [here, R¹⁶, R¹⁷ and R¹⁸ each independently represent a hydrogen atom or an organic group (for example, a methyl group, hydroxymethyl group or hydroxyethyl group, etc.)] Examples of the ethylenically unsaturated monomer having such a structure may be mentioned (meth)acrylamide, N-(hydroxymethyl) (meth)acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is an alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ each independently represent an organic group (for example, a linear or branched alkyl group having 1 to 5 carbon atoms, etc.)]. In the amino group of the present invention, a quaternized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 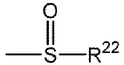 [here, R²² is an organic group (for example, an organic group having 1 to 10 carbon atoms, preferably an alkyl group having 1 to 10 carbon atoms which has one or more hydroxyl groups, etc.)].
As a polymer having such a structure, there may be mentioned a copolymer disclosed in the publication of JP 2014-48278A, etc.

As a synthesizing method of the copolymer according to the present invention, it can be synthesized by the methods of radical polymerization, anion polymerization, cation polymerization, etc., which are general synthetic methods of an acrylic polymer or a methacrylic polymer, etc. The form can be taken various kinds of methods such as solution polymerization, suspension polymerization, emulsion polymerization, bulk polymerization, etc.

As the solvent for the polymerization reaction, it may be water, phosphate-buffered saline or an alcohol such as ethanol, etc., or a mixed solvent in which these are combined, and desirably contains water or ethanol. Further, it is preferable to contain 10% by mass or more and 100% by mass or less of water or ethanol. Moreover, it is preferable to contain 50% by mass or more and 100% by mass or less of water or ethanol. Furthermore, it is preferable to contain 80% by mass or more and 100% by mass or less of water or ethanol. Still further, it is preferable to contain 90% by mass or more and 100% by mass or less of water or ethanol. It is preferable that the sum of water and ethanol is 100% by mass.

As a reaction concentration, for example, a concentration of the compound represented by the above-mentioned formula (A) or the formula (B) in the reaction solvent is preferably made 0.01% by mass to 4% by mass. If the concentration is 4% by mass or more, for example, the copolymer sometimes gelled in the reaction solvent due to strong association property possessed by the phosphate group represented by the formula (A). If the concentration is 0.01% by mass or less, a concentration of the obtained varnish is too low, so that it is difficult to prepare a coating agent for obtaining a coating film with a sufficient film thickness. The concentration is more preferably 0.01% by mass to 3% by mass, for example, 3% by mass, 2% by mass or 1% by mass.

Also, in the synthesis of the copolymer according to the present invention, for example, after preparing an acidic phosphoric acid ester monomer (half salt) described in the formula (1), polymerization may be carried out with the compound represented by the formula (C) to prepare a copolymer.

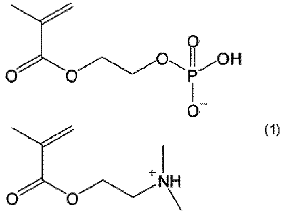

The phosphate group-containing monomer is a monomer easily associated, so that it may be added dropwise in the reaction solvent little by little so as to rapidly disperse therein when it is added dropwise into the reaction system.

Further, the reaction solvent may be heated (for example, 40°C to 100°C) so as to raise solubility of the monomer and the polymer.

In order to efficiently proceed the polymerization reaction, it is desirable to use a polymerization initiator. Examples of the polymerization initiator may be used 2,2'-azobis(isobutyronitrile), 2,2'-azobis(2-methylbutyronitrile), 2,2'-azobis(2,4-dimethylvaleronitrile) (available from Wako Pure Chemical Industries, Ltd.; V-65, 10 hour half-life temperature; 51°C), 4,4'-azobis(4-cyanovaleric acid), 2,2'-azobis(4-methoxy-2,4-dimethylvaleronitrile), 1,1'-azobis(cyclohexane-1-carbonitrile), 1-[(1-cyano-1-methylethyl)azo]formamide, 2,2'-azobis[2-(2-imidazolin-2-yl)propane] dihydrochloride (available from Wako Pure Chemical Industries, Ltd.; VA-044, 10 hour half-life temperature; 44°C), 2,2'-azobis[2-(2-imidazolin-2-yl)propane] (available from Wako Pure Chemical Industries, Ltd.; VA-061, 10 hour half-life temperature; 61°C), 2,2'-azobis(2-methylpropionamidine) dihydrochoride, 2,2'-azobis[2-methyl-N-(2-hydroxyethyl)propionamide] (available from Wako Pure Chemical Industries, Ltd.; VA-086, 10 hour half-life temperature; 86°C), benzoyl peroxide (BPO), 2,2'-azobis[N-(2-carboxyethyl)-2-methylpropionamidine] n-hydrate (available from Wako Pure Chemical Industries, Ltd.; VA-057, 10 hour half-life temperature; 57°C), 4,4'-azobis(4-cyano-pentanoic acid) (available from Wako Pure Chemical Industries, Ltd.; V-501), 2,2'-azobis[2-(2-imidazolin-2-yl)propane] disulfate dihydrate (available from Wako Pure Chemical Industries, Ltd.; VA-046B, 10 hour half-life temperature; 46°C), 2,2'-azobis(2-amidinopropane) dihydrochloride (available from Wako Pure Chemical Industries, Ltd.; V-50, 10 hour half-life temperature; 56°C), peroxydisulfuric acid or t-butylhydroperoxide, etc.

When solubility in water, ion balance and interaction with the monomer are taking into consideration, it is preferably selected from 2,2'-azobis[2-methyl-N-(2-hydroxyethyl)propionamide], 2,2'-azobis[N-(2-carboxyethyl)-2-methylpropionamidine] n-hydrate, 4,4'-azobis(4-cyanopentanoic acid), 2,2'-azobis[2-(2-imidazolin-2-yl)-propane] dihydrochloride, 2,2'-azobis[2-(2-imidazolin-2-yl)propane] disulfate dihydrate, 2,2'-azobis[2-(2-imidazolin-2-yl)propane], 2,2'-azobis(2-amidinopropane) dihydrochloride and peroxydisulfuric acid.

When solubility in an organic solvent, ion balance and interaction with the monomer are taking into consideration, it is desirable to use 2,2'-azobis(2,4-dimethylvaleronitrile) or 2,2'-azobis(isobutyronitrile).

As an amount of the polymerization initiator to be added, it is 0.05% by mass to 10% by mass based on the total weight of the monomer to be used for polymerization.

The reaction conditions are to carry out stirring in a reaction vessel by heating to 50°C to 200°C with an oil bath, etc., for 1 hour to 48 hours, more preferably at 80°C to 150°C for 5 hours to 30 hours to proceed the polymerization reaction whereby a copolymer according to the present invention can be obtained. The reaction atmosphere is preferably a nitrogen atmosphere.

As a reaction procedure, whole reaction substances may be charged in the reaction solvent at room temperature and then these may be heated to the above-mentioned temperature and polymerized, or all or a part of the mixture of the reaction substances may be added dropwise little by little into a previously heated solvent.

According to the latter reaction procedure, a varnish containing the copolymer of the present invention can be prepared by the producing method including the steps of adding a mixture containing the compounds of the above-mentioned formulae (A), (B) and (C), the solvent and the polymerization initiator dropwise into a solvent maintained at a temperature higher than the 10 hour half-life temperature of the polymerization initiator, and reacting (polymerizing) the same.

In this embodiment, the concentration of the compound represented by the above-mentioned formula (A) or the formula (B) in the reaction solvent can be adjusted to, for example, 0.01% by mass to 10% by mass by employing the above-mentioned reaction procedure and temperature conditions. In this embodiment, even if the concentration exceeds 4% by mass, the dropping phase and the reaction phase become transparent and uniform solutions before the reaction, and gelation of the copolymer after the reaction in the reaction solvent can be suppressed. Other conditions in this embodiment are the same as mentioned above.

The weight molecular weight of the copolymer according to the present invention may be about several thousands to several millions, and preferably 5,000 to 5,000,000. It is further preferably 10,000 to 2,000,000. In addition, it may be either of a random copolymer, a block copolymer or a graft copolymer, there is no particular limitation on the copolymerization reaction itself for manufacturing the copolymer, and a conventionally known method synthesizing in a liquid can be used such as polymerization utilizing radical polymerization, ionic polymerization, photopolymerization, macromer, emulsion polymerization, etc. These can be used any of the copolymers of the present invention solely or a plurality of the copolymers are mixed and the ratio thereof may be changed depending on the objective uses.

Also, the various copolymers thus produced may be a two-dimensional polymer or a three-dimensional polymer, and in a state of being dispersed in a solution containing water or an alcohol. That is, in the varnishes containing these polymers, it is not preferable to generate ununiform gelation or turbid precipitation, and is preferably a transparent varnish, a dispersed colloidal state varnish, or a sol.

The coating agent to be used for forming a coating onto the cell culture container according to the present invention may be prepared by diluting a desired copolymer with a desired solvent to a predetermined concentration, depending on the cases.

Such a solvent may be mentioned water, phosphate-buffered saline (PBS) and an alcohol. The alcohol may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, which may be used alone or a mixed solvent of a combination thereof, and from the viewpoint of dissolution of the copolymer, it is preferably selected from water, PBS, ethanol, propanol and a mixed solvent thereof, and more preferably selected from water, ethanol and a mixed solvent thereof.

Further, the coating agent may be prepared from a varnish containing the copolymer. The varnish containing the copolymer can be prepared by, for example, the producing method containing the step of reacting (polymerizing) the compounds represented by the above-mentioned formulae (A), (B) and (C) in a solvent with a total concentration of the compounds of 0.01% by mass to 20% by mass.

A concentration of the solid content in the coating agent is desirably 0.01 to 50% by mass to uniformly form a coating film. Also, a concentration of the copolymer in the coating agent is preferably 0.01 to 4% by mass, more preferably 0.01 to 3% by mass, particularly preferably 0.01 to 2% by mass, and further preferably 0.01 to 1% by mass. If the concentration of the copolymer is less than 0.01% by mass, a coating film which has a sufficient film thickness cannot be formed since the concentration of the copolymer in the coating agent is too low, while if it exceeds 4% by mass, storage stability of the coating agent becomes worse, and there is a possibility to cause precipitation or gelation of the dissolved material.

Incidentally, to the coating agent, in addition to the above-mentioned copolymer and the solvent, other substances may be added within the range that does not impair the properties of the obtainable coating, if necessary. As the other substances, there may be mentioned preservatives, surfactants, antioxidants, reductants, primers that enhance adhesive property with the substrate, fungicides and sugars, etc.

In order to adjust ion balance of the copolymer in the coating agent, a step of adjusting a pH in the coating agent in advance may be contained. Adjustment of the pH may be carried out, for example, by adding a pH adjusting agent to the composition containing the above-mentioned copolymer and the solvent and made the pH of the composition 3.5 to 9.0, preferably 3.5 to 8.5, and further preferably 4.0 to 8.0. A kind of the usable pH adjusting agent and an amount thereof can be optionally selected depending on the concentration of the above-mentioned copolymer or an existing ratio of the anion and the cation thereof, and the like.

Examples of the pH adjusting agent may be mentioned organic amines such as ammonia, diethanolamine, pyridine, N-methyl-D-glucamine, tris(hydroxymethyl)-aminomethane, etc.; alkali metal hydroxides such as potassium hydroxide, sodium hydroxide, etc.; alkali metal halides such as potassium chloride, sodium chloride, etc.; inorganic acids such as sulfuric acid, phosphoric acid, hydrochloric acid, carbonic acid, etc., or alkali metal salts thereof; quaternary ammonium cations such as choline, etc.; or a mixture thereof (for example, a buffer such as phosphate buffered saline, etc.). Among these, ammonia, diethanolamine, sodium hydroxide, choline, N-methyl-D-glucamine, tris(hydroxymethyl)aminomethane are preferable, and in particular, ammonia, diethanolamine, sodium hydroxide and choline are preferable.

In order to improve cell-non-adhesive property, phosphoric acid may be further added to the coating agent. An amount of the phosphoric acid to be added is generally, for example, 0.01% by weight to 10% by weight when the whole agent is made 100% by weight.

The cell culture container of the present invention has a coating formed by the above-mentioned coating agent at least a part of the surface of the cell culture container. Specifically, it has the coating at least a part of the surface at the inside and/or outside of the container, which can be contacted with a culture medium containing a biological substance such as cells and peptides relating to cell proliferation. In particular, among the surface at the inside surface of the container, which is capable of contacting with a culture medium which contains a biological substance such as cells and peptides relating to cell proliferation, it has a coating preferably at 50% or more, more preferably 80% or more, further preferably 90% or more, particularly preferably 100%.

The container to which the copolymer is coated, which constitutes the cell culture container of the present invention, may be containers with an optional shape capable of using in this field of the art, and may be mentioned, for example, dishes or schale generally used for cell culture such as petri dishes, dishes for tissue culture, multi dishes, etc., flasks such as a cell culture flask, a spinner flask, etc., bags such as plastic bags, Teflon (Registered Trademark) bags, culture bags, etc., plates such as microplates, microwell plates, multi plates, multiwell plates, etc., and bottles such as chamber slide, tubes, trays, roller bottles and the like. It is preferably mentioned 6 to 1536 well multiwell plates and schale.

Also, the material of the container may be used glass, a metal, a metal containing compound or a semi-metal containing compound, activated charcoal or a resin. The metal may be mentioned a typical metal: (an alkali metal: Li, Na, K, Rb, Cs; an alkaline earth metal: Ca, Sr, Ba, Ra), a magnesium group element: Be, Mg, Zn, Cd, Hg; an aluminum group element: Al, Ga, In; a rare earth element: Y, La, Ce, Pr, Nd, Sm, Eu; a tin group element: Ti, Zr, Sn, Hf, Pb, Th; an iron group element: Fe, Co, Ni; a vanadium group element: V, Nb, Ta, a chromium group element: Cr, Mo, W, U; a manganese group element: Mn, Re; a noble metal: Cu, Ag, Au; and a platinum group element: Ru, Rh, Pd, Os, Ir, Pt, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned, for example, cellulose, cellulose triacetate (CTA), nitrocellulose (NC), cellulose to which dextran sulfate has been fixed, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra high molecular weight polyethylene (UHPE), polydimethylsiloxane (PDMS), acrylonitrile-butadiene-styrene resin (ABS) or Teflon (Registered Trademark). In the manufacture of the cell culture container of the present invention, at the time of coating the copolymer to exist at least a part of the surface of the container, it is not necessary to treat it at a high temperature, so that a resin having low heat resistance, etc., can be also applied.

A material(s) of the container may be one kind or a combination of two or more kinds. Among these materials, it is preferably glass, silicon, silicon oxide, polystyrene (PS), polypropylene (PP), polyether sulfone (PES), polyethylene terephthalate (PET), polycarbonate (PC), polyvinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene (PS), polypropylene (PP) or stainless (SUS304, SUS316, SUS316L, etc.).

The present invention relates to a method for manufacturing a cell culture container having a coating onto at least a part of the surface of the container, which comprises a step of contacting the coating agent as mentioned above with at least a part of the surface of the container. There is no particular limitation on the contact of the coating agent and the surface of the container, and there may be used a method in which the container is dipped in the coating agent, the coating agent is added to the container and allowed to stand for a predetermined time, or coating the coating agent onto the surface of the container, etc., and a method of adding the coating agent to the container and allowing to stand for a predetermined time is preferable. Addition can be carried out, for example, by adding the coating agent with 0.5 to 1-fold amount of the whole volume of the container using a syringe, etc. Standing is carried out by appropriately selecting a time and a temperature depending on the material of the container and the kind of the coating agent and carried out, for example, for 5 minutes to 24 hours, preferably for 30 minutes to 3 hours, at 10 to 35°C, preferably at 20 to 30°C, and most preferably at 25°C. According to the procedure, a cell culture container having a coating onto at least a part of the surface of the container, preferably whole surface thereof can be manufactured.

Also, the coating on the surface of the container obtained by such a method can be used as a cell culture container after the step of contacting with at least a part of the above-mentioned surface of the container, preferably after the step of adding a coating agent and allowing to stand for a predetermined time, without passing through the drying step as such, or after the step of washing using water or a medium (for example, water, buffer, medium, etc.) of a sample to be applied to cell culture.

That is, after the step of contacting at least a part of the above-mentioned surface of the container, preferably after the step of adding a coating agent and allowing to stand for a predetermined time, within 48 hours, preferably within 24 hours, further preferably within 12 hours, further preferably within 6 hours, further preferably within 3 hours, further preferably within 1 hour, without passing through the drying step as such, or after washing using water or a medium (for example, water, buffer, medium, etc., particularly preferably medium (for example, DMEM medium (Dulbecco's modified Eagle medium)) of a sample to be applied to cell culture, it can be used as a cell culture container.

The container may be applied to a drying step. The drying step is carried out under atmosphere or under vacuum, preferably at a temperature in the range of -200°C to 200°C. By the drying step, the solvent in the above-mentioned coating agent is removed, and also the formula (a) and the formula (b) of the copolymer relating to the present invention are formed an ion bonding with each other to completely adhere to the substrate.

The coating can be formed, for example, by the drying at room temperature (10°C to 35°C, preferably at 20°C to 30°C, for example, 25°C), and it may be dried, for example, at 40°C to 50°C to form the coating more rapidly. In addition, a drying step by the freeze-drying method at an extremely low temperature to a low temperature (-200°C to around -30°C) may be used. The freeze drying is called as vacuum freeze-drying, and is a method in which a material to be desired to dry is cooled with a refrigerant in general and remove the solvent under a vacuum state by sublimation. A general refrigerant to be used in the freeze drying may be mentioned a mixed medium of dry ice and methanol (-78°C), liquid nitrogen (-196°C), etc.

If the drying temperature is lower than -200°C, an unusual refrigerant must be used which lacks versatility, and a long time is required for solvent sublimation so that efficiency is poor. If the drying temperature exceeds 200°C, ionic bonding reaction on the surface of the coating proceeds excessively, whereby the surface loses hydrophilicity and a function to inhibit adhesion of biological substances such as cells and peptides relating to cell proliferation, etc., is not exhibited. More preferred drying temperature is 10°C to 180°C, and more preferred drying temperature is 25°C to 150°C.

The coating of the present application is produced through the simple and easy step as mentioned above.

In addition, in order to remove impurities, unreacted monomer, etc., remained in the coating, and further, to adjust ion balance of the copolymer in the coating, it may be carried out a step of washing with at least one solvent selected from water and an aqueous solution containing an electrolyte. Washing is desirably running water washing or ultrasonic wave washing, etc. The above-mentioned water and the aqueous solution containing an electrolyte may be a material heated, for example, in the range of 40°C to 95°C. The aqueous solution containing an electrolyte is preferably PBS, physiological saline (containing only sodium chloride), Dulbecco's phosphate-buffered saline, Tris buffered physiological saline, HEPES buffered physiological saline and veronal buffered physiological saline, and particularly preferably PBS. After adhesion, the coating film does not elute even when washed with water, PBS and alcohol, etc., and still remains firmly adhered to the substrate. Even if cells or proteins are adhered to the formed coating, these can be easily removed by washing, etc., thereafter, so that the surface of the container onto which the coating of the present invention has been formed has a function to inhibit adhesion of biological substances such as cells and peptides relating to cell proliferation whereby it becomes suitable for a long term non-adhesion culture.

A film thickness of the coating applied to the surface of the container of the present invention can be appropriately adjusted according to the shape of the container or the kind of the sample, etc., and may be substantially uniform over the whole surface of the container or may be partially ununiform, which is not particularly limited, and preferably 10 to 1000Å, further preferably 10 to 500Å, and most preferably 10 to 300Å.

The present invention also relates to a method for producing a cell aggregate which comprises using the above-mentioned cell culture container. By subjecting to the above-mentioned coating onto at least a part of the surface of the cell culture container, even if the culture term is continued for a long term, adhesion of the cells to the surface of the container is inhibited so that cell aggregates can be efficiently manufactured by suspension culture, etc. The meanings of cells, cell aggregates, coatings and containers, etc., in such a method are as defined above.

### EXAMPLES

In the following, the present invention is explained in more detail based on Synthetic Examples, Preparation Examples, Examples, Test Examples, etc., but the present invention is not limited by these.

### <Synthetic Example 1>

32.10 g of pure water was added to 7.00 g of acid phosphoxyethyl methacrylate (product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 32.10 g of ethanol, 9.41 g of 2-(diethylamino)ethyl methacrylate (available from Tokyo Chemical Industry Co., Ltd.), 1.34 g of n-butyl methacrylate (available from Tokyo Chemical Industry Co., Ltd.) and 0.09 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 96.31 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 178.35 g of a copolymer-containing varnish having a solid content of about 9.74% by mass was obtained. A weight average molecular weight of the obtained transparent liquid by GFC was about 210,000.

### <Synthetic Example 2>

16.05 g of pure water was added to 7.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 48.16 g of ethanol, 9.41 g of 2-(diethylamino)ethyl methacrylate (available from Tokyo Chemical Industry Co., Ltd.), 1.34 g of n-butyl methacrylate (available from Tokyo Chemical Industry Co., Ltd.) and 0.09 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 96.31 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 178.35 g of a copolymer-containing varnish having a solid content of about 9.61%% by mass was obtained. A weight average molecular weight of the obtained transparent liquid by GFC was about 220,000.

### <Synthetic Example 3>

15.81 g of pure water was added to 7.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 47.44 g of ethanol, 9.41 g of 2-(diethylamino)ethyl methacrylate (available from Tokyo Chemical Industry Co., Ltd.), 1.07 g of ethyl methacrylate (available from Tokyo Chemical Industry Co., Ltd.) and 0.09 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 94.88 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 175.71 g of a copolymer-containing varnish having a solid content of about 9.75% by mass was obtained. A weight average molecular weight of the obtained transparent liquid by GFC was about 280,000.

### <Synthetic Example 4>

17.03 g of pure water was added to 7.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 51.08 g of ethanol, 9.41 g of 2-(diethylamino)ethyl methacrylate (available from Tokyo Chemical Industry Co., Ltd.), 2.42 g of ethyl methacrylate (available from Tokyo Chemical Industry Co., Ltd.) and 0.09 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 102.17 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 189.20 g of a copolymer-containing varnish having a solid content of about 9.52% by mass was obtained. A weight average molecular weight of the obtained transparent liquid by GFC was about 250,000.

### <Synthetic Example 5>

18.59 g of pure water was added to 7.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 55.77 g of ethanol, 9.41 g of 2-(diethylamino)ethyl methacrylate (available from Tokyo Chemical Industry Co., Ltd.), 4.14 g of ethyl methacrylate (available from Tokyo Chemical Industry Co., Ltd.) and 0.10 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 111.53 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 185.88 g of a copolymer-containing varnish having a solid content of about 9.49% by mass was obtained. A weight average molecular weight of the obtained transparent liquid by GFC was about 200,000.

### <Synthetic Example 6>

11.64 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 34.91 g of ethanol, 6.72 g of 2-(diethylamino)ethyl methacrylate (available from Tokyo Chemical Industry Co., Ltd.), 1.14 g of n-hexyl methacrylate (available from Tokyo Chemical Industry Co., Ltd.) and 0.06 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 69.82 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 129.29 g of a copolymer-containing varnish having a solid content of about 9.80% by mass was obtained. A weight average molecular weight of the obtained transparent liquid by GFC was about 210,000.

### <Synthetic Example 7>

21.13 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 21.13 g of ethanol, 5.72 g of 2-(dimethylamino)ethyl methacrylate (available from Tokyo Chemical Industry Co., Ltd.), 0.96 g of n-butyl methacrylate (available from Tokyo Chemical Industry Co., Ltd.) and 0.06 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 63.40 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 117.41 g of a copolymer-containing varnish having a solid content of about 9.56% by mass was obtained. A weight average molecular weight of the obtained transparent liquid by GFC was about 380,000.

### <Synthetic Example 8>

12.38 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 37.15 g of ethanol, 5.72 g of 2-(dimethylamino)ethyl methacrylate (available from Tokyo Chemical Industry Co., Ltd.), 2.97 g of ethyl methacrylate (available from Tokyo Chemical Industry Co., Ltd.) and 0.07 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 74.31 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 137.41 g of a copolymer-containing varnish having a solid content of about 8.89% by mass was obtained. A weight average molecular weight of the obtained transparent liquid by GFC was about 540,000.

### <Synthetic Example 9>

20.24 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 20.24 g of ethanol, 3.82 g of 2-(dimethylamino)ethyl methacrylate (available from Tokyo Chemical Industry Co., Ltd.), 2.37 g of ethyl methacrylate (available from Tokyo Chemical Industry Co., Ltd.) and 0.06 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 60.73 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 112.47 g of a copolymer-containing varnish having a solid content of about 9.34% by mass was obtained. A weight average molecular weight of the obtained transparent liquid by GFC was about 580,000.

### <Synthetic Example 10>

24.81 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 24.81 g of ethanol, 5.72 g of 2-(dimethylamino)ethyl methacrylate (available from Tokyo Chemical Industry Co., Ltd.), 2.97 g of ethyl methacrylate (available from Tokyo Chemical Industry Co., Ltd.) and 0.09 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 74.42 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 137.81 g of a copolymer-containing varnish having a solid content of about 9.38% by mass was obtained. A weight average molecular weight of the obtained transparent liquid by GFC was about 280,000.

### <Synthetic Example 11>

19.58 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 19.58 g of ethanol, 2.29 g of 2-(dimethylamino)ethyl methacrylate (available from Tokyo Chemical Industry Co., Ltd.), 2.69 g of 2-(diethylamino)ethyl methacrylate (available from Tokyo Chemical Industry Co., Ltd.), 0.84 g of n-butyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 58.73 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 108.75 g of a copolymer-containing varnish having a solid content of about 9.82% by mass was obtained. A weight average molecular weight of the obtained transparent liquid by GFC was about 330,000.

### <Synthetic Example 12>

20.54 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 20.54 g of ethanol, 1.64 g of 2-(dimethylamino)ethyl methacrylate (available from Tokyo Chemical Industry Co., Ltd.), 3.84 g of 2-(diethyl-amino)ethyl methacrylate (available from Tokyo Chemical Industry Co., Ltd.), 0.88 g of n-butyl methacrylate (available from Tokyo Chemical Industry Co., Ltd.) and 0.06 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 61.61 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 114.09 g of a copolymer-containing varnish having a solid content of about 9.81% by mass was obtained. A weight average molecular weight of the obtained transparent liquid by GFC was about 320,000.

### <Synthetic Example 13>

18.74 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 18.74 g of ethanol, 2.86 g of 2-(dimethylamino)ethyl methacrylate (available from Tokyo Chemical Industry Co., Ltd.), 1.68 g of 2-(diethyl-amino)ethyl methacrylate (available from Tokyo Chemical Industry Co., Ltd.), 0.68 g of ethyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 56.21 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 104.09 g of a copolymer-containing varnish having a solid content of about 9.75% by mass was obtained. A weight average molecular weight of the obtained transparent liquid by GFC was about 370,000.

### <Synthetic Example 14>

20.22 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 20.22 g of ethanol, 1.64 g of 2-(dimethylamino)ethyl methacrylate (available from Tokyo Chemical Industry Co., Ltd.), 3.84 g of 2-(diethyl-amino)ethyl methacrylate (available from Tokyo Chemical Industry Co., Ltd.), 0.70 g of ethyl methacrylate (available from Tokyo Chemical Industry Co., Ltd.) and 0.06 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 60.67 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 112.36 g of a copolymer-containing varnish having a solid content of about 9.71% by mass was obtained. A weight average molecular weight of the obtained transparent liquid by GFC was about 410,000.

### <Synthetic Example 15>

18.45 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 18.45 g of ethanol, 2.86 g of 2-(dimethylamino)ethyl methacrylate (available from Tokyo Chemical Industry Co., Ltd.), 1.68 g of 2-(diethyl-amino)ethyl methacrylate (available from Tokyo Chemical Industry Co., Ltd.), 0.65 g of ethyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 57.83 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 107.09 g of a copolymer-containing varnish having a solid content of about 9.70% by mass was obtained.
A weight average molecular weight of the obtained transparent liquid by GFC was about 490,000.

### <Comparative Synthetic Example 1>

While cooling 25.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)) at 35°C or lower, 29.95 g of choline (48-50% aqueous solution: available from Tokyo Chemical Industry Co., Ltd.) was added thereto and the mixture was stirred until the mixture became uniform. To the mixed solution were successively added 20.95 g of 80% aqueous methacroylcholine chloride solution (available from Tokyo Chemical Industry Co., Ltd.), 28.67 g of n-butyl methacrylate (available from Tokyo Chemical Industry Co., Ltd.), 0.70 g of 2,2'-azobis(2,4-dimethylvaleronitrile) (available from Wako Pure Chemical Industries, Ltd.; V-65) and 110.84 g of ethanol while maintaining to 35°C or lower. Further, an aqueous solution in which 0.70 g of 2,2'-azobis(N-(2-carboxyethyl)-2-methylpropion-amidine) n-hydrate (available from Wako Pure Chemical Industries, Ltd.; VA-057) had been dissolved in 27.71 g of pure water was added to the above-mentioned solution while maintaining to 35°C or lower, and a mixed solution in which all the above-mentioned materials were contained which became uniform by sufficiently stirring was introduced into a dropping funnel. On the other hand, 56.81 g of pure water and 131.62 g of ethanol were charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 1 hour. After the dropping, the mixture was stirred under heating for 24 hours while maintaining the above-mentioned circumstance to obtain 432.97 g of a copolymer-containing varnish having a solid content of about 19.86% by mass was obtained. A weight average molecular weight of the obtained transparent liquid by GFC was about 19,000.

### <Comparative Synthetic Example 2>

While cooling 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)) at 35°C or lower, 5.99 g of choline (48-50% aqueous solution: available from Tokyo Chemical Industry Co., Ltd.) was added thereto and the mixture was stirred until the mixture became uniform. To the mixed solution were successively added 4.19 g of methacroylcholine chloride 80% aqueous solution (available from Tokyo Chemical Industry Co., Ltd.), 16.16 g of methyl methacrylate (available from Tokyo Chemical Industry Co., Ltd.), 0.25 g of 2,2'-azobis(2,4-dimethylvaleronitrile)(available from Wako Pure Chemical Industries, Ltd.; V-65) and 35.78 g of ethanol while maintaining to 35°C or lower. Further, an aqueous solution in which 0.25 g of 2,2'-azobis(N-(2-carboxyethyl)-2-methylpropionamidine) n-hydrate (available from Wako Pure Chemical Industries, Ltd.; VA-057) had been dissolved in 8.95 g of pure water was added to the above-mentioned solution while maintaining to 35°C or lower, and a mixed solution in which all the above-mentioned materials were contained which became uniform by sufficiently stirring was introduced into a dropping funnel. On the other hand, 20.72 g of pure water and 42.49 g of ethanol were charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 1 hour. After the dropping, the mixture was stirred under heating for 4 hours while maintaining the above-mentioned circumstance to obtain 112.16 g of a copolymer-containing varnish having a solid content of about 20.34% by mass was obtained. A weight average molecular weight of the obtained transparent liquid by GFC was about 26,000.

### <Comparative Synthetic Example 3>

While cooling 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)) at 35°C or lower, 5.99 g of choline (48-50% aqueous solution: available from Tokyo Chemical Industry Co., Ltd.) was added thereto and the mixture was stirred until the mixture became uniform. To the mixed solution were successively added 4.19 g of 80% aqueous methacroylcholine chloride solution (available from Tokyo Chemical Industry Co., Ltd.), 10.75 g of ethyl methacrylate (available from Tokyo Chemical Industry Co., Ltd.), 0.19 g of 2,2'-azobis(2,4-dimethylvaleronitrile) (available from Wako Pure Chemical Industries, Ltd.; V-65) and 28.71 g of ethanol while maintaining to 35°C or lower. Further, an aqueous solution in which 0.19 g of 2,2'-azobis(N-(2-carboxyethyl)-2-methylpropionamidine) n-hydrate (available from Wako Pure Chemical Industries, Ltd.; VA-057) had been dissolved in 7.18 g of pure water was added to the above-mentioned solution while maintaining to 35°C or lower, and a mixed solution in which all the above-mentioned materials were contained which became uniform by sufficiently stirring was introduced into a dropping funnel. On the other hand, 15.86 g of pure water and 34.10 g of ethanol were charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 1 hour. After the dropping, the mixture was stirred under heating for 4 hours while maintaining the above-mentioned circumstance to obtain 168.47 g of a copolymer-containing varnish having a solid content of about 20.02% by mass was obtained. A weight average molecular weight of the obtained transparent liquid by GFC was about 37,000.

### <Comparative Synthetic Example 4>

30.32 g of pure water was added to 8.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 30.32 g of ethanol, 8.76 g of 2-(diethylamino)ethyl methacrylate (available from Tokyo Chemical Industry Co., Ltd.) and 0.08 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 90.97 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 168.47 g of a copolymer-containing varnish having a solid content of about 9.61% by mass was obtained. A weight average molecular weight of the obtained transparent liquid by GFC was about 290,000.

### <Comparative Synthetic Example 5>

18.05 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 18.05 g of ethanol, 2.29 g of 2-(dimethylamino)ethyl methacrylate (available from Tokyo Chemical Industry Co., Ltd.), 2.69 g of 2-(diethylamino)ethyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 54.15 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 100.28 g of a copolymer-containing varnish having a solid content of about 9.70% by mass was obtained. A weight average molecular weight of the obtained transparent liquid by GFC was about 950,000.

### <Comparative Synthetic Example 6>

18.95 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 18.95 g of ethanol, 1.64 g of 2-(dimethylamino)ethyl methacrylate (available from Tokyo Chemical Industry Co., Ltd.), 3.84 g of 2-(diethylamino)ethyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 56.85 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 105.29 g of a copolymer-containing varnish having a solid content of about 9.98% by mass was obtained. A weight average molecular weight of the obtained transparent liquid by GFC was about 500,000.

### <Comparative Synthetic Example 7>

17.26 g of pure water was added to 5.00 g of acid phosphoxyethyl methacrylate (the product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)), and the mixture was sufficiently dissolved. Then, 17.26 g of ethanol, 2.86 g of 2-(dimethylamino)ethyl methacrylate (available from Tokyo Chemical Industry Co., Ltd.), 1.68 g of 2-(diethylamino)ethyl methacrylate (available from Tokyo Chemical Industry Co., Ltd.) and 0.05 g of 2,2'-azobis(N-(2-carboxyethyl)-2-methylpropionamidine) n-hydrate (available from Wako Pure Chemical Industries, Ltd.; VA-057) were successively added to the aqueous solution of Phosmer M while maintaining to 20°C or lower. A mixed solution in which all the above-mentioned materials were contained which became uniform by sufficiently stirring was introduced into a dropping funnel. On the other hand, 51.79 g of pure water was charged in a three-necked flask equipped with a cooling tube separately and subjected to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 2 hours. After the dropping, the mixture was stirred under heating for 4 hours while maintaining the above-mentioned circumstance to obtain 95.90 g of a copolymer-containing varnish having a solid content of about 9.98% by mass was obtained. A weight average molecular weight of the obtained transparent liquid by GFC was about 600,000.

### (Preparation of silicon wafer)

A commercially available silicon wafer for evaluating a semiconductor was used as such.

### (Preparation of QCM sensor (PS))

An Au-deposited quartz oscillator (Q-Sense,QSX304) was washed for 10 minutes by using a UV/ozone washing apparatus (UV253E, manufactured by Filgen, Inc.), and immediately thereafter immersed in a solution in which 0.0772 g of 2-aminoethanethiol (available from Tokyo Chemical Industry Co., Ltd.) had been dissolved in 1,000 mL of ethanol for 24 hours. After washing the surface of the sensor with ethanol, it was naturally dried, and a varnish in which 1.00 g of polystyrene (available from Aldrich Co.) had been dissolved in 99.00 g of toluene was spin coated onto a side of a membrane sensor with 3,500 rpm/30 sec and dried at 120°C for 1 minutes to prepare a QCM sensor (PS).

### <Preparation Example 1>

To 4.00 g of the varnish containing the copolymer obtained in the above-mentioned Synthetic Example 1 were added 24.0 g of pure water, 10.88 g of ethanol and 0.27 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 79Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 2>

To 3.60 g of the varnish containing the copolymer obtained in the above-mentioned Synthetic Example 1 were added 14.28 g of pure water, 17.03 g of ethanol and 0.15 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 51Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 3>

To 3.60 g of the varnish containing the copolymer obtained in the above-mentioned Synthetic Example 1 were added 14.43 g of pure water and 17.05 g of ethanol, and the mixture was sufficiently stirred to prepare a coating agent. A pH thereof was 6.7. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 39Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 4>

To 3.80 g of the varnish containing the copolymer obtained in the above-mentioned Synthetic Example 2 were added 22.19 g of pure water, 9.82 g of ethanol, 0.34 g of 1 mol/L aqueous sodium hydroxide solution (IN) (available from KANTO CHEMICAL CO., INC.) and 0.18 g of an aqueous phosphoric acid solution diluted to 5% with pure water (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 62Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 5>

To 3.49 g of the varnish containing the copolymer obtained in the above-mentioned Synthetic Example 3 were added 21.34 g of pure water, 9.19 g of ethanol and 0.18 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 59Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 6>

To 3.50 g of the varnish containing the copolymer obtained in the above-mentioned Synthetic Example 4 were added 20.71 g of pure water, 8.95 g of ethanol and 0.19 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 72Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 7>

To 3.51 g of the varnish containing the copolymer obtained in the above-mentioned Synthetic Example 5 were added 20.66 g of pure water, 8.88 g of ethanol and 0.18 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 99Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 8>

To 3.51 g of the varnish containing the copolymer obtained in the above-mentioned Synthetic Example 5 were added 13.52 g of pure water, 16.16 g of ethanol and 0.11 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 62Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 9>

To 3.51 g of the varnish containing the copolymer obtained in the above-mentioned Synthetic Example 5 were added 13.63 g of pure water and 16.16 g of ethanol, and the mixture was sufficiently stirred to prepare a coating agent. A pH thereof was 6.8. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 51Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 10>

To 3.51 g of the varnish containing the copolymer obtained in the above-mentioned Synthetic Example 6 were added 21.43 g of pure water, 9.28 g of ethanol and 0.19 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 110Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 11>

To 3.51 g of the varnish containing the copolymer obtained in the above-mentioned Synthetic Example 7 were added 19.17 g of pure water, 8.30 g of ethanol and 0.22 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 75Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 12>

To 3.60 g of the varnish containing the copolymer obtained in the above-mentioned Synthetic Example 7 were added 20.93 g of pure water, 9.52 g of ethanol and 0.19 g of 1 mol/L aqueous sodium hydroxide solution (IN) (available from KANTO CHEMICAL CO., INC.), and the mixture was sufficiently stirred to prepare a coating agent. A pH thereof was 7.2. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 98Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 13>

To 5.80 g of the varnish containing the copolymer obtained in the above-mentioned Synthetic Example 7 were added 34.23 g of pure water and 15.42 g of ethanol, and the mixture was sufficiently stirred to prepare a coating agent. A pH thereof was 6.2. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 52Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 14>

To 3.51 g of the varnish containing the copolymer obtained in the above-mentioned Synthetic Example 8 were added 19.17 g of pure water, 8.30 g of ethanol and 0.22 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 50Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 15>

To 3.60 g of the varnish containing the copolymer obtained in the above-mentioned Synthetic Example 8 were added 21.36 g of pure water, 9.22 g of ethanol and 0.17 g of 1 mol/L aqueous sodium hydroxide solution (IN) (available from KANTO CHEMICAL CO., INC.), and the mixture was sufficiently stirred to prepare a coating agent. A pH thereof was 7.3. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 72Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 16>

To 3.60 g of the varnish containing the copolymer obtained in the above-mentioned Synthetic Example 8 were added 21.12 g of pure water, 9.22 g of ethanol and 0.41 g of 1 mol/L aqueous sodium hydroxide solution (IN) (available from KANTO CHEMICAL CO., INC.), and the mixture was sufficiently stirred to prepare a coating agent. A pH thereof was 8.0. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 60Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 17>

To 4.00 g of the varnish containing the copolymer obtained in the above-mentioned Synthetic Example 9 were added 22.6 g of pure water, 10.40 g of ethanol and 0.47 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 65Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 18>

To 3.70 g of the varnish containing the copolymer obtained in the above-mentioned Synthetic Example 10 were added 21.10 g of pure water, 9.70 g of ethanol and 0.23 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 69Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 19>

To 3.61 g of the varnish containing the copolymer obtained in the above-mentioned Synthetic Example 10 were added 20.93 g of pure water, 9.26 g of ethanol and 0.13 g of 1 mol/L aqueous sodium hydroxide solution (IN) (available from KANTO CHEMICAL CO., INC.), and the mixture was sufficiently stirred to prepare a coating agent. A pH thereof was 7.0. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 45Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 20>

To 3.60 g of the varnish containing the copolymer obtained in the above-mentioned Synthetic Example 10 were added 7.12 g of pure water, 22.77 g of ethanol and 0.30 g of 1 mol/L aqueous sodium hydroxide solution (IN) (available from KANTO CHEMICAL CO., INC.), and the mixture was sufficiently stirred to prepare a coating agent. A pH thereof was 8.0. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 61Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 21>

To 3.60 g of the varnish containing the copolymer obtained in the above-mentioned Synthetic Example 10 were added 6.76 g of pure water, 22.87 g of ethanol and 0.75 g of 1 mol/L aqueous sodium hydroxide solution (IN) (available from KANTO CHEMICAL CO., INC.), and the mixture was sufficiently stirred to prepare a coating agent. A pH thereof was 9.0. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 170Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 22>

To 3.60 g of the varnish containing the copolymer obtained in the above-mentioned Synthetic Example 10 were added 6.76 g of pure water, 22.87 g of ethanol and 0.75 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 63Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 23>

To 3.60 g of the varnish containing the copolymer obtained in the above-mentioned Synthetic Example 10 were added 13.63 g of pure water, 16.19 g of ethanol and 0.54 g of 1 mol/L aqueous sodium hydroxide solution (IN) (available from KANTO CHEMICAL CO., INC.), and the mixture was sufficiently stirred to prepare a coating agent. A pH thereof was 8.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 140Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 24>

To 3.61 g of the varnish containing the copolymer obtained in the above-mentioned Synthetic Example 10 were added 14.19 g of pure water, 16.04 g of ethanol and 0.23 g of 1 mol/L aqueous sodium hydroxide solution (IN) (available from KANTO CHEMICAL CO., INC.), and the mixture was sufficiently stirred to prepare a coating agent. A pH thereof was 7.5. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 84Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 25>

To 3.60 g of the varnish containing the copolymer obtained in the above-mentioned Synthetic Example 10 were added 20.77 g of pure water, 9.37 g of ethanol and 0.27 g of 1 mol/L aqueous sodium hydroxide solution (IN) (available from KANTO CHEMICAL CO., INC.), and the mixture was sufficiently stirred to prepare a coating agent. A pH thereof was 7.6. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 52Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 26>

To 3.60 g of the varnish containing the copolymer obtained in the above-mentioned Synthetic Example 10 were added 13.76 g of pure water, 16.00 g of ethanol and 0.25 g of 1 mol/L aqueous sodium hydroxide solution (IN) (available from KANTO CHEMICAL CO., INC.), and the mixture was sufficiently stirred to prepare a coating agent. A pH thereof was 7.7. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 44Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 27>

To 3.70 g of the varnish containing the copolymer obtained in the above-mentioned Synthetic Example 11 were added 21.98 g of pure water, 10.13 g of ethanol and 0.34 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 149Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 28>

To 3.70 g of the varnish containing the copolymer obtained in the above-mentioned Synthetic Example 12 were added 21.99 g of pure water, 10.11 g of ethanol and 0.31 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 101Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 29>

To 3.70 g of the varnish containing the copolymer obtained in the above-mentioned Synthetic Example 13 were added 21.74 g of pure water, 10.05 g of ethanol and 0.39 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 136Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 30>

To 3.70 g of the varnish containing the copolymer obtained in the above-mentioned Synthetic Example 14 were added 21.72 g of pure water, 10.00 g of ethanol and 0.30 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 40Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Preparation Example 31>

To 3.70 g of the varnish containing the copolymer obtained in the above-mentioned Synthetic Example 15 were added 21.62 g of pure water, 10.00 g of ethanol and 0.39 g of 1 mol/L aqueous sodium hydroxide solution (IN) (available from KANTO CHEMICAL CO., INC.), and the mixture was sufficiently stirred to prepare a coating agent. A pH thereof was 7.4. The obtained coating agent was subjected to spin coating onto the above-mentioned silicon wafer at 1,500 rpm/60 sec, and dried in an oven at 50°C for 24 hours. Thereafter, the uncured coating agent attached onto the coating film was sufficiently washed with PBS and pure water to obtain a silicon wafer onto which a coating film had been formed. When a film thickness of the coating film was confirmed by using the above-mentioned silicon wafer with a spectroscopic ellipsometer, it was 73Å.

Also, the above-mentioned coating agent was spin coated onto a QCM sensor with 3,500 rpm/30 sec, and baked in an oven at 50°C for 24 hours as a drying step. Thereafter, the excessively attached uncured coating agent was washed with PBS and ultrapure water each twice as a washing step to obtain a surface-treated QCM sensor (PS).

### <Comparative Preparation Example 1>

The above-mentioned QCM sensor (PS) was used as such.

### <Comparative Preparation Example 2>

To 3.30 g of the varnish containing the copolymer obtained in the above-mentioned Comparative Synthetic Example 1 were added 8.28 g of pure water, 20.22 g of ethanol and 0.41 g of 1 mol/L hydrochloric acid (IN) (available from KANTO CHEMICAL CO., INC.), and the mixture was sufficiently stirred to prepare a coating agent. A pH thereof was 3.4. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 584Å.

### <Comparative Preparation Example 3>

To 7.01 g of the varnish containing the copolymer obtained in the above-mentioned Comparative Synthetic Example 2 were added 18.66 g of pure water, 45.03 g of ethanol and 0.78 g of 1 mol/L hydrochloric acid (IN) (available from KANTO CHEMICAL CO., INC.), and the mixture was sufficiently stirred to prepare a coating agent. A pH thereof was 3.3. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 715Å.

### <Comparative Preparation Example 4>

To 7.00 g of the varnish containing the copolymer obtained in the above-mentioned Comparative Synthetic Example 3 were added 18.10 g of pure water, 44.17 g of ethanol and 0.88 g of 1 mol/L hydrochloric acid (IN) (available from KANTO CHEMICAL CO., INC.), and the mixture was sufficiently stirred to prepare a coating agent. A pH thereof was 3.3. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 659Å.

### <Comparative Preparation Example 5>

To 380.10 g of the varnish containing the copolymer obtained in the above-mentioned Comparative Synthetic Example 4 were added 2,229.37 g of pure water, 1,079.67 g of ethanol and 64.02 g of 1 mol/L aqueous sodium hydroxide solution (IN) (available from KANTO CHEMICAL CO., INC.), and the mixture was sufficiently stirred to prepare a coating agent. A pH thereof was 7.4. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 187Å.

### <Comparative Preparation Example 6>

To 380.10 g of the varnish containing the copolymer obtained in the above-mentioned Comparative Synthetic Example 5 were added 2,229.37 g of pure water, 1,079.67 g of ethanol and 64.02 g of 1 mol/L aqueous sodium hydroxide solution (IN) (available from KANTO CHEMICAL CO., INC.), and the mixture was sufficiently stirred to prepare a coating agent. A pH thereof was 7.4. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 255Å.

### <Comparative Preparation Example 7>

To 380.10 g of the varnish containing the copolymer obtained in the above-mentioned Comparative Synthetic Example 6 were added 2,229.37 g of pure water, 1079.67 g of ethanol and 64.02 g of 1 mol/L aqueous sodium hydroxide solution (IN) (available from KANTO CHEMICAL CO., INC.), and the mixture was sufficiently stirred to prepare a coating agent. A pH thereof was 7.4. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 47Å.

### <Comparative Preparation Example 8>

To 380.10 g of the varnish containing the copolymer obtained in the above-mentioned Comparative Synthetic Example 7 were added 2,229.37 g of pure water, 1,079.67 g of ethanol and 64.02 g of 1 mol/L aqueous sodium hydroxide solution (IN) (available from KANTO CHEMICAL CO., INC.), and the mixture was sufficiently stirred to prepare a coating agent. A pH thereof was 7.4. In the same method as in Preparation Example 1, a silicon wafer onto which a coating film had been formed and a surface-treated QCM sensor (PS) were obtained. When a film thickness of the coating film was confirmed by using the above-mentioned silicon wafer with a spectroscopic ellipsometer, it was 64Å.

### <Test Example 1>

### [Protein adhesion test; QCM-D measurement]

The surface-treated QCM sensor (PS) obtained by each Preparation Example and Comparative Example was attached to a dissipation type quartz oscillator microbalance QCM-D(E4, Q-Sense), and PBS was flown until a stabilized baseline in which change in frequency became 1 Hz or less with 1 hour was established. Next, PBS was flown about 10 minutes as a frequency of the stabilized baseline being 0 Hz. Subsequently, a solution to which 15 wt% bovine serum (FBS), L-Glutamine, and penicillin and streptomycin as antibiotics had been added was flown to 41010 - Basal Medium Eagle (BME), no Glutamine (available from Thermo Fisher Scientific Co., Ltd.) for 30 minutes, thereafter a shift (Δf) of an adsorption induced frequency of a ninth overtone after flowing PBS again for about 20 minutes was read. For analysis, Q-Tools (Q-Sense) were used, and the value of the shift (Δf) of an adsorption induced frequency converted into a mass (ng/cm²) per a unit surface area with a shift (Δf) of an adsorption induced frequency explained by the Sauerbrey equation is shown as an adhered amount of a biological substance which is shown in Table 1. Preparation Example showed an adsorption amount of various kinds of proteins with lower by one digit.

**[Table 1]**

| | |
|---|---|
| Preparation Example 1 | 9 (ng/cm²) |
| Preparation Example 2 | Detection limit or less |
| Preparation Example 3 | Detection limit or less |
| Preparation Example 4 | 1 (ng/cm²) |
| Preparation Example 5 | 22 (ng/cm²) |
| Preparation Example 6 | Detection limit or less |
| Preparation Example 7 | 45 (ng/cm²) |
| Preparation Example 8 | 12 (ng/cm²) |
| Preparation Example 9 | 42 (ng/cm²) |
| Preparation Example 10 | 12 (ng/cm²) |
| Preparation Example 11 | 16 (ng/cm²) |
| Preparation Example 12 | Detection limit or less |
| Preparation Example 13 | Detection limit or less |
| Preparation Example 14 | Detection limit or less |
| Preparation Example 15 | 20 (ng/cm²) |
| Preparation Example 16 | 28 (ng/cm²) |
| Preparation Example 17 | Detection limit or less |
| Preparation Example 18 | Detection limit or less |
| Preparation Example 19 | Detection limit or less |
| Preparation Example 20 | 10 (ng/cm²) |
| Preparation Example 21 | 36 (ng/cm²) |
| Preparation Example 22 | Detection limit or less |
| Preparation Example 23 | 13 (ng/cm²) |
| Preparation Example 24 | Detection limit or less |
| Preparation Example 25 | 41 (ng/cm²) |
| Preparation Example 26 | 31 (ng/cm²) |
| Preparation Example 27 | 10 (ng/cm²) |
| Preparation Example 28 | Detection limit or less |
| Preparation Example 29 | 4 (ng/cm²) |
| Preparation Example 30 | 66 (ng/cm²) |
| Preparation Example 31 | 53 (ng/cm²) |
| | |
| Comparative Preparation Example 1 | 566 (ng/cm²) |
| Comparative Preparation Example 5 | 208 (ng/cm²) |
| Comparative Preparation Example 6 | 198 (ng/cm²) |
| Comparative Preparation Example 7 | 231 (ng/cm²) |
| Comparative Preparation Example 8 | 272 (ng/cm²) |

### <Example 1>

The following respective treatment steps were carried out in this order to prepare cell culture containers in the present invention.
Treatment 1: The varnish containing the copolymer prepared in the above-mentioned Preparation Example 7 was filtered through a filter having a mesh size of 0.22 µm, and then, added to a well of 96 well cell culture plate (manufactured by BD Biosciences, #351172) so that it became 200 µL (solid content: 1% by mass)/well, and after allowing to stand at room temperature for 1 hour, excess varnish was removed.
Treatment 2: By using an oven (manufactured by Advantec Toyo Kaisha Ltd., dryer FC-612), the material was dried at 50°C overnight. Thereafter, 200 µL of sterilized water was added per well, and then, removed to carry out washing. In the same manner, washing was further carried out twice to obtain a coated cell culture container.

### <Example 2>

In the same manner as in Example 1, except for changing the varnish containing the copolymer prepared in Preparation Example 7 used in Example 1 to the varnish containing the copolymer prepared in Preparation Example 8, a coated cell culture container was obtained.

### <Example 3>

In the same manner as in Example 1, except for changing the varnish containing the copolymer prepared in Preparation Example 7 used in Example 1 to the varnish containing the copolymer prepared in Preparation Example 9, a coated cell culture container was obtained.

### <Example 4>

In the same manner as in Example 1, except for changing the varnish containing the copolymer prepared in Preparation Example 7 used in Example 1 to the varnish containing the copolymer prepared in Preparation Example 10, a coated cell culture container was obtained.

### <Example 5>

In the same manner as in Example 1, except for changing the varnish containing the copolymer prepared in Preparation Example 7 used in Example 1 to the varnish containing the copolymer prepared in Preparation Example 11, a coated cell culture container was obtained.

### <Example 6>

In the same manner as in Example 1, except for changing the varnish containing the copolymer prepared in Preparation Example 7 used in Example 1 to the varnish containing the copolymer prepared in Preparation Example 14, a coated cell culture container was obtained.

### <Example 7>

In the same manner as in Example 1, except for changing the varnish containing the copolymer prepared in Preparation Example 7 used in Example 1 to the varnish containing the copolymer prepared in Preparation Example 15, a coated cell culture container was obtained.

### <Example 8>

In the same manner as in Example 1, except for changing the varnish containing the copolymer prepared in Preparation Example 7 used in Example 1 to the varnish containing the copolymer prepared in Preparation Example 16, a coated cell culture container was obtained.

### <Example 9>

In the same manner as in Example 1, except for changing the varnish containing the copolymer prepared in Preparation Example 7 used in Example 1 to the varnish containing the copolymer prepared in Preparation Example 18, a coated cell culture container was obtained.

### <Example 10>

In the same manner as in Example 1, except for changing the varnish containing the copolymer prepared in Preparation Example 7 used in Example 1 to the varnish containing the copolymer prepared in Preparation Example 19, a coated cell culture container was obtained.

### <Example 11>

In the same manner as in Example 1, except for changing the varnish containing the copolymer prepared in Preparation Example 7 used in Example 1 to the varnish containing the copolymer prepared in Preparation Example 20, a coated cell culture container was obtained.

### <Example 12>

In the same manner as in Example 1, except for changing the varnish containing the copolymer prepared in Preparation Example 7 used in Example 1 to the varnish containing the copolymer prepared in Preparation Example 21, a coated cell culture container was obtained.

### <Example 13>

In the same manner as in Example 1, except for changing the varnish containing the copolymer prepared in Preparation Example 22 used in Example 1 to the varnish containing the copolymer prepared in Preparation Example 8, a coated cell culture container was obtained.

### <Example 14>

In the same manner as in Example 1, except for changing the varnish containing the copolymer prepared in Preparation Example 7 used in Example 1 to the varnish containing the copolymer prepared in Preparation Example 23, a coated cell culture container was obtained.

### <Example 15>

In the same manner as in Example 1, except for changing the varnish containing the copolymer prepared in Preparation Example 7 used in Example 1 to the varnish containing the copolymer prepared in Preparation Example 24, a coated cell culture container was obtained.

### <Comparative Example 1>

In the same manner as in Example 1, except for changing the varnish containing the copolymer prepared in Preparation Example 7 used in Example 1 to the varnish containing the copolymer prepared in Comparative Preparation Example 2, a coated cell culture container was obtained.

### <Comparative Example 2>

The following respective treatment steps were carried out in this order to prepare cell culture containers in the present invention.
Treatment 1: The varnish containing the copolymer prepared in the above-mentioned Comparative Preparation Example 3 was filtered through a filter having a mesh size of 0.22 µm, and then, added to a well of 96 well cell culture plate (manufactured by BD Biosciences, #351172) so that it became 200 µL (solid content: 2% by mass)/well, and after allowing to stand at room temperature for 1 hour, excess varnish was removed.
Treatment 2: By using an oven (manufactured by Advantec Toyo Kaisha Ltd., dryer FC-612), the material was dried at 50°C overnight. Thereafter, 200 µL of sterilized water was added per well, and then, removed to carry out washing. In the same manner, washing was further carried out twice to obtain a coated cell culture container.

### <Comparative Example 3>

In the same manner as in Comparative Example 2, except for changing the varnish containing the copolymer prepared in Comparative Preparation Example 3 used in Comparative Example 2 to the varnish containing the copolymer prepared in Comparative Preparation Example 4, a coated cell culture container was obtained.

### <Comparative Example 4>

In the same manner as in Example 1, except for changing the varnish containing the copolymer prepared in Preparation Example 7 used in Example 1 to the varnish containing the copolymer prepared in Comparative Preparation Example 5, a coated cell culture container was obtained.

### <Comparative Example 5>

In the same manner as in Example 1, except for changing the varnish containing the copolymer prepared in Preparation Example 7 used in Example 1 to the varnish containing the copolymer prepared in Comparative Preparation Example 6, a coated cell culture container was obtained.

### <Test Example 2: fibroblast cell adhesion inhibition test >

### (Preparation of coating plate)

The coated cell culture containers obtained by Examples 1 to 15 and Comparative Examples 1 to 5 were used. As a sample of a positive control, a commercially available cell low-adhesion plate (available from Corning Co., #3474) was used. As a negative control, a 96 well cell culture plate (available from BD Biosciences, #351172) to which no coating had been applied was used.

### (Preparation of cells)

As the cells, mouse embryonic fibroblasts C3H10T1/2 (available from DS Pharma Biomedical Co., Ltd.) were used. The medium used for culture of the cells was a BME medium (available from Thermo Fisher Scientific Co., Ltd.) containing 10 (v/v) % FBS (available from Corning Co.) and an L-glutamine-penicillin-streptomycin stabilizing solution (available from Sigma-Aldrich Corporation). The cells were statically cultured for 2 days or longer using a schale (petri dish) (8 mL of medium) having a diameter of 10 cm while in a state of maintaining a 5 (v/v) % carbon dioxide concentration in a 37°C/CO₂ incubator. Subsequently, the present cells were washed with 5 mL of PBS, then, 1 mL of trypsin-EDTA solution (available from Invitrogen) was added thereto, and the cells were detached and suspended in 10 mL of the above-mentioned medium, respectively. This suspension was centrifuged (manufactured by Tommy Seiko Co., Ltd., model number LC-200, 1,000 rpm/3 minutes, room temperature), then, the supernatant was removed and the above-mentioned medium was added to prepare a cell suspension.

### (Cell adhesion test)

To each plate prepared as mentioned above, 100 µL of each cell suspension was added so as to be 2×10³ cells/well. Thereafter, the samples were allowed to stand in a CO₂ incubator at 37°C for 4 days in the state of maintaining a 5% carbon dioxide concentration.

### (Observation of cell adhesion)

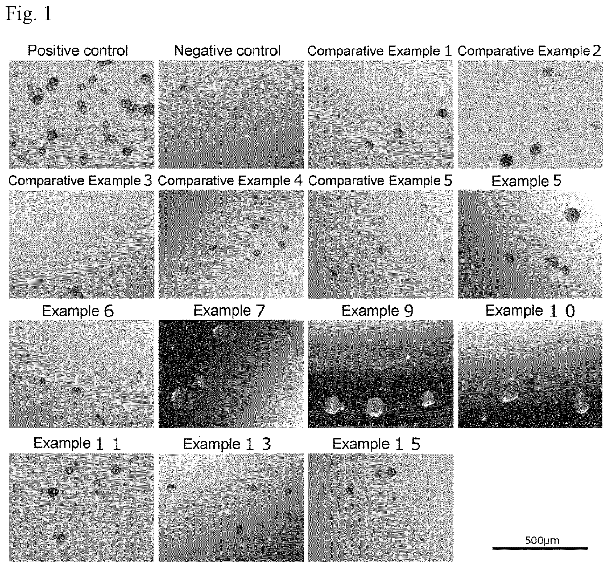

Four days after the culture, adhesion of the cells to the plate of each Example and Comparative Example and the plates of positive control and negative control were compared based on observation (magnification: 4-fold and 10-fold) with an inverted microscope (manufactured by Olympus Corporation, IX73). The results of each plate (4 days after the culture) are shown in Fig. 1. Also, 10 µL of a Cell Counting Kit-8 solution (available from DOJINDO Laboratories) was added per one well, and allowed to stand in a CO₂ incubator at 37°C for 2 hours. Thereafter, an absorbance at 450 nm was measured by an absorbance meter (manufactured by Molecular Devices, SpectraMax). Each measured value was obtained by subtracting therefrom the measured value in the well to which the, respectively. The results are shown in Table 2.

**[Table 2]**

| Absorbance measurement results | |
|---|---|
| | average |
| Positive control | 0.040 |
| Negative control | 1.052 |
| Comparative Example 1 | 0.080 |
| Comparative Example 2 | 0.129 |
| Comparative Example 3 | 0.078 |
| Comparative Example 4 | 0.386 |
| Comparative Example 5 | 0.190 |
| Example 5 | 0.059 |
| Example 6 | 0.056 |
| Example 7 | 0.052 |
| Example 9 | 0.032 |
| Example 10 | 0.037 |
| Example 11 | 0.035 |
| Example 13 | 0.038 |
| Example 15 | 0.037 |

As mentioned above, it was shown that whereas in Comparative Examples 1 to 5 and negative control plate, cells were adhered, in Examples 5 to 7, 9 to 11, 13, 15 and positive control plate, cells were not adhered. At this time, the cells which had not been adhered formed cell aggregates (spheroid).

### <Test Example 3: Cancer cell adhesion inhibiting test>

### (Preparation of coating plate)

The coated cell culture containers obtained by Examples 1 to 15 and Comparative Examples 1 to 5 were used. As a sample of a positive control, a commercially available cell low-adhesion plate (available from Corning Co., #3474) was used. As a negative control, a 96 well cell culture plate (manufactured by BD Biosciences, #351172) to which no coating had been applied was used.

### (Preparation of cells)

As the cells, human alveolar basal epithelial adenocarcinoma cell line A549 (available from DS Pharma Biomedical Co., Ltd.) were used. The medium used for culture of the cells was a DMEM (available from Wako Pure Chemical Industries, Ltd.) containing 10 (v/v) % FBS (manufactured by Corning Co.). The cells were statically cultured for 2 days or longer using a schale (petri dish) (8 mL of medium) having a diameter of 10 cm while in a state of maintaining a 5 (v/v) % carbon dioxide concentration in a 37°C/CO₂ incubator. Subsequently, the present cells were washed with 5 mL of PBS, then, 1 mL of trypsin-EDTA solution (available from Invitrogen) was added thereto, and the cells were detached and suspended in 10 mL of the above-mentioned medium, respectively. This suspension was centrifuged (manufactured by Tommy Seiko Co., Ltd., model number LC-200, 1,000 rpm/3 minutes, room temperature), then, the supernatant was removed and the above-mentioned medium was added to prepare a cell suspension.

### (Cell adhesion test)

To each plate prepared as mentioned above, 100 µL of each cell suspension was added so as to be 2×10³ cells/well. Thereafter, the samples were allowed to stand in a CO₂ incubator at 37°C for 21 days in the state of maintaining a 5% carbon dioxide concentration.

### (Observation of cell adhesion)

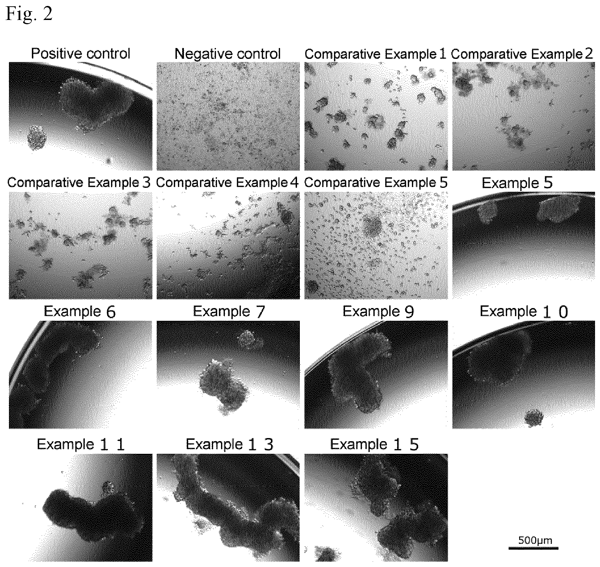

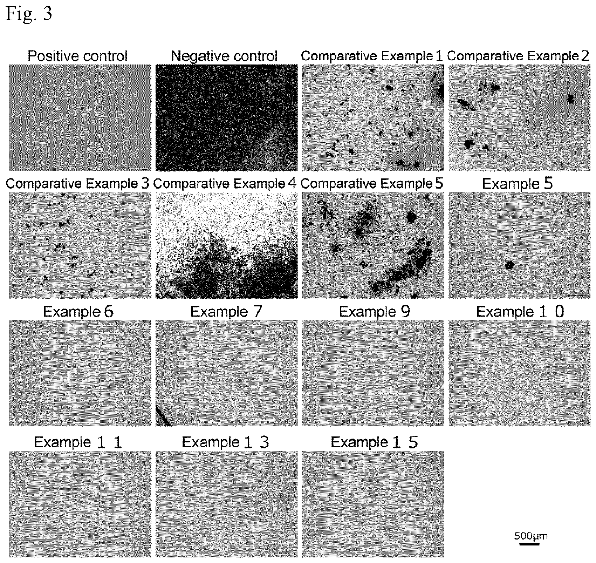

21 days after the culture, adhesion of the cells to each plate was compared based on observation (magnification: 4-fold) with an inverted microscope (manufactured by Olympus Corporation, IX73). The results of each plate (21 days after the culture) are shown in Fig. 2. After observation, the medium was removed, and 50 µL of a staining solution dissolved in sterilized water so as to contain 0.5 (w/v) % crystal violet (available from Wako Pure Chemical Industries, Ltd.) and 20 (v/v) % methanol (available from Wako Pure Chemical Industries, Ltd.) was added to each well, and staining was carried out for 10 minutes. Thereafter, 200 µL of sterilized water was added per well, and then, removed to carry out washing. Similarly, washing was further carried out twice. Adhesion of the cells to each plate was compared based on observation (magnification: 4-fold) with an inverted microscope (manufactured by Olympus Corporation, IX73). The results of each plate are shown in Fig. 3.

Before staining, spheroids were formed on the plates of Examples 5 to 7, 9 to 11, 13 and 15 and the positive control, but in the plates of Comparative Examples 1 to 5 and the negative control, adhesion of the cells was observed and no spheroid was formed. After staining, adhesion of stained cells was almost not observed in the plates of Examples 5 to 7, 9 to 11, 13 and 15 and the positive control, but in the plates of Comparative Examples 1 to 5 and the negative control, adhesion of stained cells was observed.

### <Examples 16 to 20, Comparative Example 6>

In the same manner as in Examples 2, 7, 9, 11 and 13 and Comparative Example 1 except for changing the 96 well cell culture plate (manufactured by BD Biosciences, #351172) used in Examples 2, 7, 9, 11 and 13 and Comparative Example 1 to a 96 well plate (manufactured by Corning Co., #3363 (material: polypropylene)), coated plates of Examples 16 to 20 and Comparative Example 6 were obtained, respectively. As a negative control, a 96 well cell culture plate to which no coating had been applied was used.

### <Examples 21 to 25, Comparative Example 7>

In the same manner as in Examples 2, 7, 9, 11 and 13 and Comparative Example 1 except for changing the 96 well cell culture plate (manufactured by BD Biosciences, #351172) used in Examples 2, 7, 9, 11 and 13 and Comparative Example 1 to a 96 well plate (manufactured by Corning Co., #9017 (material: polystyrene)), coated plates of Examples 21 to 25 and Comparative Example 7 were obtained, respectively. As a negative control, a 96 well cell culture plate to which no coating had been applied was used.

### <Test Example 4: Protein adsorption inhibiting test>

### (Preparation of coating plate)

The coated plates obtained in Examples 16 to 25 and Comparative Examples 6 to 7 were used. As a negative control, a 96 well cell culture plate to which no coating had been applied was used.

### (Protein adsorption experiment)

Horseradish peroxidase (abbreviated to as HRP) labelled Goat Anti-Mouse IgG (available from SoutherBioteck) was diluted by D-PBS(-) (available from Wako Pure Chemical Industries, Ltd.), charged in a well coated as mentioned above, and allowed to stand at room temperature for 30 minutes. After washing with D-PBS(-), TMB 1-Component Microwell Peroxidase Substrate, SureBlue (available from Kirkegaard & Perry Laboratories, Inc., abbreviated to as TMB) was added to react with HRP, and TMB Stop Solution (available from Kirkegaard & Perry Laboratories, Inc.) was added to stop the reaction. The optical density (450 nm) of the TMB solution at this time was measured by a plate reader (SPECTRAMAX 190, Molecular Devices), and evaluated to as a protein adsorbed amount. The results are shown in Table 3.

**[Table 3]**

| Absorbance measurement results | | | |
|---|---|---|---|
| | Corning #3363 (PP) | | Corning #9017 (PS) |
| Negative control | 0.916 | Negative control | 0.362 |
| Comparative Example 6 | 0.024 | Comparative Example 7 | 0.019 |
| Example 16 | 0.042 | Example 21 | 0.017 |
| Example 17 | 0.028 | Example 22 | 0.017 |
| Example 18 | 0.011 | Example 23 | 0.008 |
| Example 19 | 0.015 | Example 24 | 0.011 |
| Example 20 | 0.027 | Example 25 | 0.014 |

In the coated plates made of a polypropylene and a polystyrene of Examples 16 to 25 and Comparative Examples 6 to 7, it was confirmed that they have a function of inhibiting adhesion of proteins.

### <Test Example 5: Cell adhesion inhibiting test to PDMS>

### (Preparation of PDMS plate)

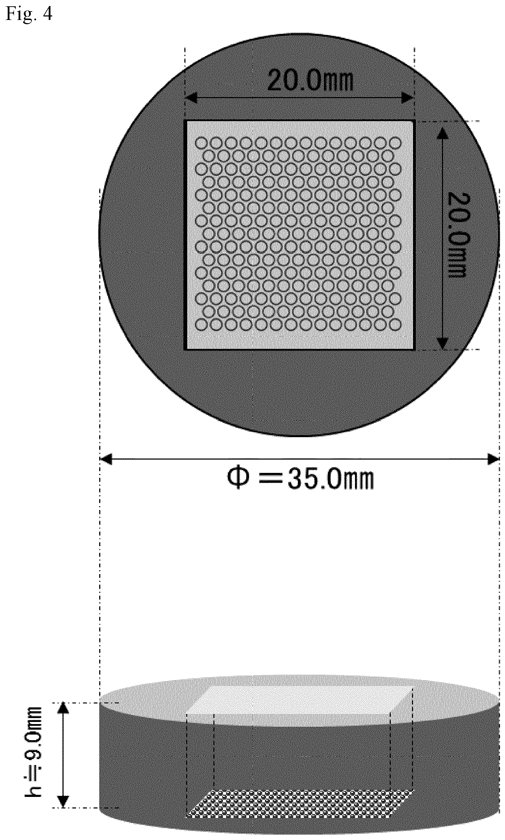

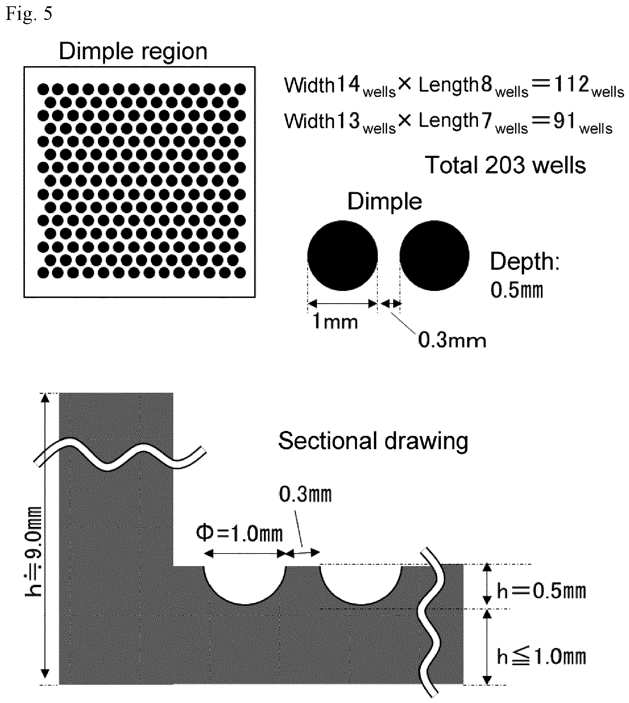

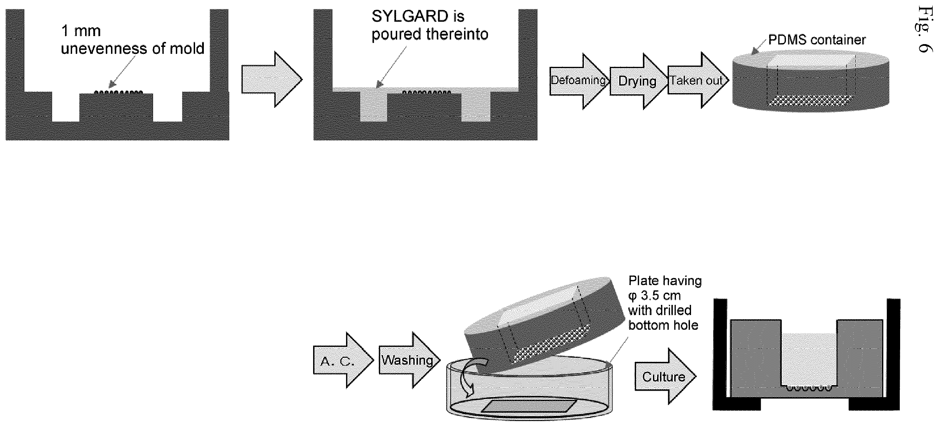

A mold which can transfer the shape of Fig. 4 is prepared. With a ratio of 10.00 g of a main agent of SYLGARD 184 silicone elastomer (available from Dow Corning) and 1.00 g of a curing agent, these are mixed, stirred and poured into the prepared mold. After defoaming with a vacuum pump, it is dried in an oven at 80°C for 1 hour. After cooling to room temperature, a cured product of PDMS is taken out from the mold. The cured product is immersed in pure water, and an autoclave sterilization treatment is carried out at 120°C for 60 minutes. The bottom of ASNOL schale (petri dish): ϕ40 × 13.5 mm (manufactured by AS ONE Corporation) is cut in comply with the dimple region in Fig. 5 with 20 mm square, and the PDMS cured product washed with 70% ethanol is placed in the petri dish and capped. It is made a PDMS coating plate having a dimple number of 203, where a diameter of 1 mm (a distance between dimples of 0.3 mm), a depth of 0.5 mm, and a volume per one dimple of about 0.3 µL (see Fig. 6).

### (Preparation of coating plate)

Into separate plates of the PDMS coating plates prepared as mentioned above was injected each 1 mL of the coating agent and 3% pHEMA (polyhydroxyethyl methacrylate) solution used in Examples 9 and 11. These were allowed to stand for 1 hour, and then, removed and dried in an oven at 50°C for 24 hours. Thereafter, the coated wells were washed with 1 mL of pure water each with three times and used in the test as Examples 26 and 27 and Comparative Example 8, respectively. As a negative control, a well not applied the coating was used.

### (Preparation of cells)

As the cells, mouse embryonic fibroblasts C3H10T1/2 (available from DS Pharma Biomedical Co., Ltd.) were used. The medium used for culture of the cells was a BME medium (available from Thermo Fisher Scientific Co., Ltd.) containing 10 (v/v) % FBS (available from Corning Co.) and an L-glutamine-penicillin-streptomycin stabilizing solution (available from Sigma-Aldrich Corporation). The cells were statically cultured for 2 days or longer using a schale (petri dish) (8 mL of medium) having a diameter of 10 cm while in a state of maintaining a 5 (v/v) % carbon dioxide concentration in a 37°C/CO₂ incubator. Subsequently, the present cells were washed with 5 mL of PBS, then, 1 mL of trypsin-EDTA solution (available from Invitrogen) was added thereto, and the cells were detached and suspended in 10 mL of the above-mentioned medium, respectively. This suspension was centrifuged (manufactured by Tommy Seiko Co., Ltd., model number LC-200, 1,000 rpm/3 minutes, room temperature), then, the supernatant was removed and the above-mentioned medium was added to prepare a cell suspension.

### (Cell adhesion test)

To each plate prepared as mentioned above, 1 mL of each cell suspension was added so as to be 30×10⁴ cells/plate. Thereafter, the samples were allowed to stand in a CO₂ incubator at 37°C for 2 days in the state of maintaining a 5% carbon dioxide concentration.

### (Observation of cell adhesion)

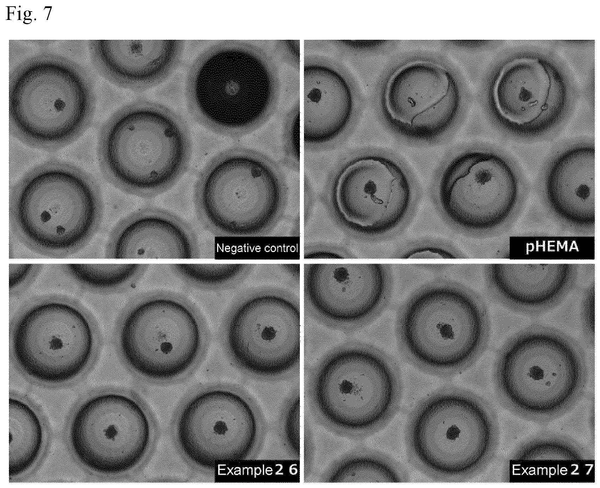

One day after the culture, adhesion of the cells to the plate of each Example and Comparative Example and the plates of positive control and negative control were compared based on observation (magnification: 4-fold) with an inverted microscope (manufactured by Olympus Corporation, IX73). The results of each plate (1 day after the culture) are shown in Fig. 7. With regard to coatability of the film, cell adhesion and the presence or absence of air bubbles after addition of the medium, the results are shown in Table 4. With regard to cell adhesion inhibition, the case where the cells were attached was indicated by ×, and the case where no cells was attached was indicated by o. With regard to coatability, the case where an image derived from the coating material which could be confirmed in an observation area other than the cells or air bubbles could be confirmed was indicated by × as coating failure, and the case where the same image as that of the negative control was maintained was indicated by o.

**[Table 4]**

| | Cell adhesion inhibition Spheroid formation | Coatability |
|---|---|---|
| Negative control | × | - |
| Comparative Example 8 (pHEMA) | × | × |
| Example 26 (Coating agent of Example 9) | ○ | ○ |
| Example 27 (Coating agent of Example 11) | ○ | ○ |

In the plates of pHEMA and negative control, cells were adhered. In Examples 26 and 27, good coatability was confirmed, and good spheroids without adhesion of the cells were confirmed.

## Claims

1. A cell culture container for long term non-adherent cell culture which comprises having a coating containing a copolymer which is a copolymer having a recurring unit which contains a group represented by the following formula (a), a recurring unit which contains a group represented by the following formula (b), and a recurring unit which contains a group represented by the following formula (c): 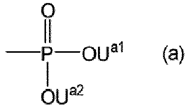 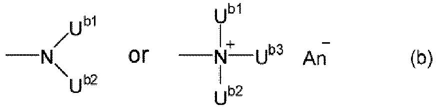
-R^{c} (c)
wherein,
U^{a1}, U^{a2}, U^{b1}, U^{b2} and U^{b3} each independently represent a hydrogen atom or a linear or branched alkyl group having 1 to 5 carbon atoms;
R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms, where the aryl portion may be substituted by a linear or branched alkyl group having 1 to 5 carbon atoms which may be substituted by a halogen atom(s); and
An⁻ represents an anion selected from the group consisting of a halide ion, an inorganic acid ion, a hydroxide ion and an isothiocyanate ion, and
a molar ratio of the recurring unit which contains a group represented by the formula (c) being 1 to 50 mol% based on the whole copolymer, provided with at least a part of a surface thereof.

2. The cell culture container according to Claim 1, wherein the recurring units containing groups represented by the above-mentioned formulae (a), (b) and (c) are recurring unit derived from monomers represented by the following formulae (A), (B) and (C): 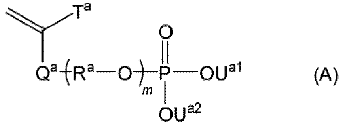 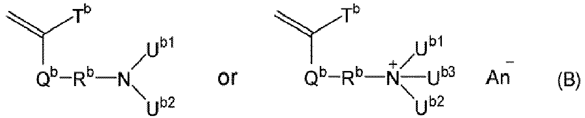 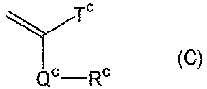 wherein,
T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms;
Q^{a} and Q^{b} each independently represent a single bond, an ester bond or an amide bond, Q^{c} represents a single bond, an ether bond or an ester bond;
R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms, where the aryl portion may be substituted by a linear or branched alkyl group having 1 to 5 carbon atoms which may be substituted by a halogen atom(s)); An⁻ represents an anion selected from the group consisting of a halide ion, an inorganic acid ion, a hydroxide ion and an isothiocyanate ion; and
m represents an integer of 0 to 6,
respectively.

3. The cell culture container according to Claim 1 or 2, wherein the copolymer further contains a crosslinking structure derived from a monomer represented by the following formula (D) or (E): 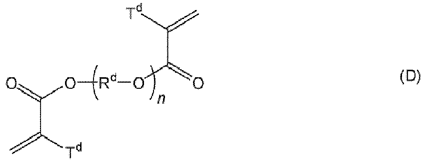 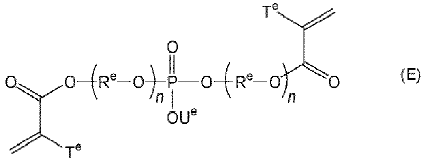 wherein,
T^{d}, T^{e} and U^{e} each independently represent a hydrogen atom or a linear or branched alkyl group having 1 to 5 carbon atoms,
R^{d} and R^{e} each independently represent a linear or branched alkylene group having 1 to 10 carbon atoms which may be substituted by a halogen atom(s); and
n represents an integer of 1 to 6.

4. The cell culture container according to any one of Claims 1 to 3, which has a function of inhibiting adhesion of a biological substance.

5. A method for manufacturing a cell culture container for long term non-adherent cell culture which comprises a step of coating a copolymer containing a recurring unit which contains a group represented by the following formula (a), a recurring unit which contains a group represented by the following formula (b), and a recurring unit which contains a group represented by the following formula (c): 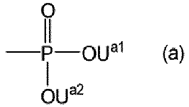 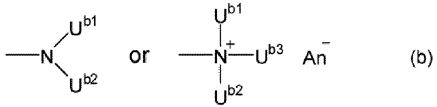
-R^{c} (c)
wherein,
U^{a1}, U^{a2}, U^{b1}, U^{b2} and U^{b3} each independently represent a hydrogen atom or a linear or branched alkyl group having 1 to 5 carbon atoms;
R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms, where the aryl portion may be substituted by a linear or branched alkyl group having 1 to 5 carbon atoms which may be substituted by a halogen atom(s); and
An⁻ represents an anion selected from the group consisting of a halide ion, an inorganic acid ion, a hydroxide ion and an isothiocyanate ion,
and a molar ratio of the recurring unit which contains a group represented by the formula (c) being 1 to 50 mol% based on a whole copolymer, onto at least a part of a surface of the container.

6. The cell culture container according to Claim 1, wherein the recurring units containing groups represented by the above-mentioned formulae (a), (b) and (c) are recurring units derived from monomers represented the following formulae (A), (B) and (C): 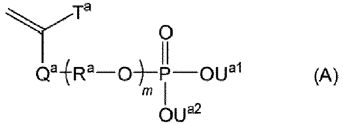 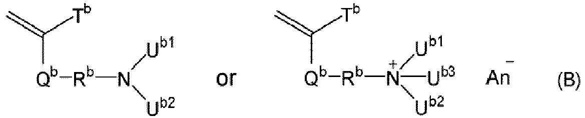 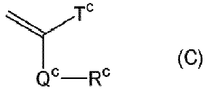 wherein,
T^{a}, T^{b}, T^{c}, U^{a1}, U^{a2}, U^{b1}, U^{b2} and U^{b3} each independently represent a hydrogen atom or a linear or branched alkyl group having 1 to 5 carbon atoms;
Q^{a} and Q^{b} each independently represent a single bond, an ester bond or an amide bond, Q^{c} represents a single bond, an ether bond or an ester bond;
R^{a} and R^{b} each independently represent a linear or branched alkylene group having 1 to 10 carbon atoms which may be substituted by a halogen atom(s), R^{c} represents a linear or branched alkyl group having 1 to 10 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms, where the aryl portion may be substituted by a linear or branched alkyl group having 1 to 5 carbon atoms which may be substituted by a halogen atom(s);
An⁻ represents an anion selected from the group consisting of a halide ion, an inorganic acid ion, a hydroxide ion and an isothiocyanate ion; and
m represents an integer of 0 to 6,
respectively.

7. The manufacturing method according to Claim 5 or 6, wherein the coating step is carried out by using a varnish containing the copolymer.

8. The manufacturing method according to Claim 7, wherein a pH of the varnish containing the copolymer is adjusted in advance.

9. The manufacturing method according to any one of Claims 5 to 8, which further comprises a step of washing the coated cell culture container before and/or after a drying step.

10. The manufacturing method according to Claim 9, wherein washing after the drying step is carried out by using at least one solvent selected from the group consisting of water and an aqueous solution containing an electrolyte(s).

11. A method for manufacturing cell aggregates which comprises using the cell culture container according to any one of Claims 1 to 4 or the cell culture container manufactured by the manufacturing method according to any one of Claims 5 to 10.
